# EUROPÄISCHE PATENTANMELDUNG

(11) **EP 4 524 596 A1**
(43) Veröffentlichungstag der Anmeldung: **19.03.2025**
(21) Anmeldenummer: 23196898.3
(22) Anmeldetag: 12.09.2023
(51) Int. Cl.: G01R 33/561, G01R 33/54, G01R 33/56, A61B 6/00, A61B 6/03, A61B 6/50

(54) **TECHNIK ZUR OPTIMIERUNG EINES UNTERABTASTUNGSFAKTORS**

(71) Anmelder: Siemens Healthineers AG, 91301 Forchheim (DE)
(72) Erfinder: Berger, Moritz, 91052 Erlangen (DE)
(74) Vertreter: Siemens Healthineers Patent Attorneys

(57) **Zusammenfassung**

Ein computer-implementiertes Verfahren (100) zur Bestimmung eines Unterabtastungsfaktors für eine medizinische Aufnahme mittels eines Tomographen umfasst ein Empfangen (S102) einer Anfrage hinsichtlich einer Planung der medizinischen Aufnahme mittels des Tomographen. Die Anfrage umfasst einen Hinweis auf einen Unterabtastungsfaktor. Eine Gruppe von Atlanten wird aus einer Bibliothek und basierend auf der empfangenen (S102) Anfrage ausgewählt (S104). Jeder Atlas umfasst einen synthetischen Messdatensatz zu einer vorbestimmten Tomographen-Modalität. Für jeden Atlas aus der ausgewählten (S104) Gruppe von Atlanten werden zwei Rekonstruktionen simuliert (S106) zum Erstellen jeweils eines Bilddatensatzes. Eine erste Rekonstruktion basiert auf dem vollständigen synthetischen Messdatensatz. Eine zweite Rekonstruktion basiert auf einem Anteil des synthetischen Messdatensatzes entsprechend des empfangenen (S102) Hinweises auf den Unterabtastungsfaktor. Eine Qualitätsmetrik der zweiten Rekonstruktion wird bestimmt (S108), wobei die simulierte (S106) zweite Rekonstruktion mit der simulierten (S106) ersten Rekonstruktion verglichen wird. Die bestimmte (S108) Qualitätsmetrik wird ausgegeben (S110) zum Festlegen des Unterabtastungsfaktors für die medizinische Aufnahme.

## Beschreibung

Die vorliegende Erfindung betrifft eine Technik zur Bestimmung eines Unterabtastungsfaktors für eine medizinische Aufnahme mittels eines Tomographen. Insbesondere werden ein Verfahren, eine Rechenvorrichtung, ein System und ein Computerprogramm bereitgestellt.

Medizinische Tomographieaufnahmen (z. B. mittels MRT oder CT) können durch Auslassen von Messschritten beschleunigt werden, um z. B. Zeit oder Dosis einzusparen. Die unterabgetasteten Daten müssen mit speziellen, angepassten Methoden rekonstruiert werden (z. B. iterative Rekonstruktion statt gefilterte Rückprojektion und/oder GRAPPA).

Derzeit wird versucht, über Deep Learning-Rekonstruktionstechniken die Bildberechnung zu verbessern. Der Algorithmus wird mit vielen Datensätzen trainiert und erzielt dann oftmals bessere Ergebnisse als die bisherigen Verfahren. Wird die Beschleunigung bei konventionellen Verfahren zu hoch eingestellt, wird das mathematische Problem numerisch instabil und Bildartefakten treten auf. Radiologinnen und Radiologen sind diese gewöhnt und können entsprechend korrumpierte Bilder erkennen. Ein großer Unterschied bei Deep Learning zu konventionellen Verfahren kann jedoch sein, dass die rekonstruierten Bilder trotz zu hoher Unterabtastung rein visuell gut aussehen bzw. für die Diagnostik scheinbar ausreichende Qualität aufweisen, jedoch entscheidende Details inklusive ganzer Läsionen nicht mehr sichtbar sind. Die bisher bekannten Artefakte durch unzureichende Daten für die Rekonstruktion sind bei Deep Learning-Rekonstruktion nicht mehr vorhanden, wie von A. Radmanesh et al. in dem Artikel "Exploring the Acceleration Limits of Deep Learning Variational Network-based Two-dimensional Brain MRI", Radiology: Artificial Intelligence 2022; 4(6):e210313, beschrieben, und der Bildeindruck lässt keinen Rückschluss mehr auf die wahrheitsgetreue Abbildung zu.

Konventionell wird durch Vorexperimente eine maximal zulässige Beschleunigung festgelegt, die der Benutzer nicht überschreiten kann. Dieser Ansatz ist jedoch nur bedingt sinnvoll, da das Potential für Beschleunigung auch z. B. von der Auflösung und der Art des Bildes abhängt. Eine pauschale Limitation eines Parameters in der MRT stellt daher immer eine untere Grenze dar. Potentiell ist es in manchen Anwendungen möglich, von höherer Beschleunigung zu profitieren, aber das konventionelle System lässt diese Wahl durch zu konservative, fest eingestellte oder bestenfalls auf Heuristiken basierende Werte nicht zu.

Es ist daher ein Ziel der vorliegenden Erfindung, eine Lösung für ressourcensparende (insbesondere hinsichtlich Aufnahmezeit, benötigte Energien und/oder Frequenzen, und/oder Strahlendosen) und gleichzeitig sinngetreue medizinische Tomographie-Aufnahme bereitzustellen. Alternativ oder ergänzend besteht die objektive Aufgabe, einen Unterabtastungsfaktor für eine medizinische Aufnahme mittels eines Tomographen derart zu optimieren, dass relevante anatomische Strukturen, insbesondere Läsionen, noch korrekt wiedergegeben werden, ohne dass spezielle vorherige Testmessungen durchgeführt werden müssen.

Diese Aufgabe wird gelöst durch ein Verfahren zur Bestimmung eines Unterabtastungsfaktors für eine medizinische Aufnahme mittels eines Tomographen, durch eine Rechenvorrichtung, durch ein System umfassend die Rechenvorrichtung und durch ein Computerprogramm (auch als Computerprogrammprodukt bezeichnet) gemäß den beigefügten unabhängigen Ansprüchen. Vorteilhafte Aspekte, Merkmale und Ausführungsformen sind in den abhängigen Ansprüchen und in der folgenden Beschreibung zusammen mit Vorteilen beschrieben.

Im Folgenden wird die erfindungsgemäße Lösung in Bezug auf das beanspruchte Verfahren zur Bestimmung eines Unterabtastungsfaktors für eine medizinische Aufnahme mittels eines Tomographen sowie in Bezug auf die beanspruchte Rechenvorrichtung beschrieben. Merkmale, Vorteile oder alternative Ausführungsformen hierin können den anderen beanspruchten Gegenständen (z. B. dem Computerprogramm oder einem Computerprogrammprodukt) zugeordnet werden und umgekehrt. Mit anderen Worten: Die Ansprüche für die Rechenvorrichtung und/oder das System können durch Merkmale verbessert werden, die im Zusammenhang mit dem Verfahren beschrieben oder beansprucht werden und umgekehrt. In diesem Fall werden die funktionalen Merkmale des Verfahrens durch strukturelle Einheiten (z. B. Schnittstellen und/oder Module) der Rechenvorrichtung und/oder des Systems verkörpert und umgekehrt.

Gemäß einem Verfahrensaspekt ist ein (insbesondere computer-implementiertes) Verfahren zur Bestimmung eines Unterabtastungsfaktors für eine medizinische Aufnahme mittels eines Tomographen bereitgestellt. Das Verfahren umfasst einen Schritt des Empfangens einer Anfrage hinsichtlich einer Planung einer medizinischen Aufnahme mittels eines Tomographen. Die Anfrage umfasst einen Hinweis auf einen Unterabtastungsfaktor. Das Verfahren umfasst ferner einen Schritt des Auswählens einer Gruppe von Atlanten aus einer Bibliothek. Jeder Atlas in der Bibliothek umfasst einen synthetischen Messdatensatz zu einer vorbestimmten Tomographen-Modalität (beispielsweise Magnetresonanztomographie, MRT). Beispielsweise kann der synthetische Messdatensatz jedes Atlas einer vollständigen Abtastung entsprechen. Das Auswählen der Gruppe von Atlanten basiert auf der empfangenen Anfrage hinsichtlich der Planung der medizinischen Aufnahme mittels des Tomographen (beispielsweise im Hinblick auf eine aufzunehmende anatomische Struktur). Das Verfahren umfasst ferner einen Schritt des Simulierens, für jeden Atlas aus der ausgewählten Gruppe von Atlanten, von mindestens zwei Rekonstruktionen zum Erstellen jeweils eines Bilddatensatzes (z. B. entsprechend dem Simulieren von mindestens zwei Messungen, insbesondere mit unterschiedlichen Unterabtastungsfaktoren und/oder ohne Unterabtastungsfaktor). Eine erste Rekonstruktion basiert auf dem vollständigen synthetischen Messdatensatz. Mindestens eine zweite Rekonstruktion basiert auf einem Anteil des synthetischen Messdatensatzes entsprechend des mit der Anfrage empfangenen Hinweises auf den Unterabtastungsfaktor. Das Verfahren umfasst ferner einen Schritt des Bestimmens einer Qualitätsmetrik der mindestens einen zweiten Rekonstruktion (insbesondere für jeden Atlas aus der ausgewählten Gruppe von Atlanten, und/oder als Mittelwert derselben). Das Bestimmen der Qualitätsmetrik umfasst ein Vergleichen der simulierten mindestens einen zweiten Rekonstruktion mit der simulierten ersten Rekonstruktion (insbesondere für jeden Atlas aus der ausgewählten Gruppe von Atlanten). Das Verfahren umfasst ferner einen Schritt des Ausgebens der bestimmten Qualitätsmetrik zum Festlegen des Unterabtastungsfaktors für die medizinische Aufnahme.

Der Unterabtastungsfaktor kann auch als Grad der Unterabtastung bezeichnet werden. Alternativ oder ergänzend kann eine Anwendung eines Unterabtastungsfaktors als Unterabtastung, Auslassen von Messschritten und/oder als Beschleunigung (z. B. der Messung und/oder medizinischen Aufnahme) bezeichnet werden. Weiterhin alternativ oder ergänzend kann der Unterabtastungsfaktor einen Faktor größer als eins umfassen, der (insbesondere invers) proportional zum Anteil der ausgelassenen Messschritte (und/oder möglichen aufnehmbaren Messdaten) ist.

Mittels des Unterabtastungsfaktors (der auch Beschleunigungsfaktor bezeichnet werden kann) und/oder des Auslassens von Messschritten kann eine für die medizinische Aufnahme benötigte Zeit (z. B. bei Magnetresonanztomographie, MRT) eingespart und/oder eine für die medizinische Aufnahme Strahlendosis (z. B. bei Computertomographie, CT) reduziert werden. Alternativ oder ergänzend kann durch den Unterabtastungsfaktor und/oder das Auslassen von Messschritten Energie eingespart werden.

Problematisch an der Unterabtastung kann die Notwendigkeit sein, nicht-aufgenommene Messwerte für eine Rekonstruktion (z. B. mathematisch) aus einer Teilmenge der aufgenommenen Messwerte zu erzeugen und/oder zu interpolieren. Während bei zu hoher Beschleunigung in herkömmlichen Rekonstruktionsverfahren beobachtbare numerische Instabilitäten und Bildartefakten auftreten, können in gegenwärtigen Rekonstruktionsverfahren, die auf Künstlicher Intelligenz (KI) basieren, nicht anhand des medizinischen Bilds ersichtliche Fehler, Glättungen und Verwischungen auftreten, aufgrund derer anatomische Strukturen, insbesondere Läsionen oder anderweitige Anomalien, nicht (oder zumindest nicht korrekt) dargestellt werden.

Mittels der erfindungsgemäßen Technik wird es ermöglicht, einen hohen (insbesondere maximalen) Unterabtastungsfaktor zu bestimmen, bei dem die relevanten anatomischen Strukturen und/oder Läsionen in einer geplanten medizinischen Aufnahme noch korrekt dargestellt werden. Diese Bestimmung kann insbesondere ohne das Ausführen einer Vielzahl von realen Messungen (z. B. im Gegensatz zu "in silico" Experimenten und/oder Simulationen), realen Vorexperimenten bzw. Testmessungen am Tomographen erfolgen.

Durch die Bestimmung des (z. B. maximalen) Unterabtastungsfaktors mittels der erfindungsgemäßen Technik kann somit eine Zeit am Tomographen optimal reduziert werden (beispielsweise indem das Verfahren zur Bestimmung des Unterabtastungsfaktors nicht direkt am Tomographen ausgeführt wird), ohne dass die Bildqualität der medizinischen Aufnahme unter eine für ihre bestimmungsgemäße Verwendung zur Diagnose und/oder zur optischen Führung während eines medizinischen Eingriffs vorbestimmte Schwelle fällt. Alternativ oder ergänzend wird durch die erfindungsgemäße Technik eine Rekonstruktion in Echtzeit (auch als "online" bezeichnet) verbessert.

Kürzere Messzeiten (z. B. 8-22 Sekunden pro Schnittbild, und/oder insgesamt 2-6 Minuten) können zu einer Verbesserung des Patienten-Wohlbefindens, einer verbesserten Zugänglichkeit des Scanners, einer verbesserte Scan-Qualität für Patienten, die nicht lange stillhalten können (z. B. Kinder), zu einer Verringerung von Beruhigungen und Betäubungen, zu einer Verringerung von Artefakten aufgrund von Bewegungen und/oder allgemein zu einer Verbesserung der Qualität von diagnostischen Bildern und/oder Bildsequenzen beitragen.

Die medizinische Aufnahme (die auch als Bilddaten oder Bild bezeichnet werden kann) kann einen vorbestimmten Bereich eines Körpers eines Patienten umfassen, beispielsweise einen Kopfbereich, einen (insbesondere Knie- und/oder Hüft-) Gelenkbereich, und/oder einen Rumpfbereich.

Alternativ oder ergänzend kann die Anfrage hinsichtlich der Planung der medizinischen Aufnahme einen Hinweis auf eine Gewebeart, eine anatomische Struktur (kurz auch: Anatomie) und/oder eine Anomalie umfassen, insbesondere einen (z. B. auf der Aufnahme erwarteten) Epilepsieherd und/oder eine Läsion, z. B. einen Tumor.

Eine Anomalie, Läsion, Tumor und/oder ein Epilepsieherd können kollektiv als Pathologie bezeichnet werden.

Eine Pathologie und/oder Läsion im Bereich des Kopfes kann beispielsweise diffuse Tumoren, eine arteriovenöse Malformation (AVM), eine Lissenzephalie und/oder ein Hirnödem umfassen.

Die Anfrage hinsichtlich der Planung der medizinischen Aufnahme kann einen Hinweis auf einen Untersuchungsbereich (auch als Untersuchungsregion bezeichnet) und/oder einen Bereich von Interesse (fachsprachlich: Region of Interest; kurz: ROI) umfassen. Der Untersuchungsbereich kann beispielsweise den Kopf, Knie, und/oder Abdomen eines Patienten umfassen. Alternativ oder ergänzend kann ein Untersuchungsbereich und/oder eine ROI eine Position einer vermuteten Pathologie und/oder Läsion, z. B. im Bereich des Kopfes, umfassen.

Alternativ oder ergänzend kann eine ROI einen Bereich in einem Bild umfassen, der (z. B. mathematisch) ausgewertet wird. Eine ROI kann einen Kreis, einen Quader, und/oder eine Kugel in einem Datensatz umfassen, aus welcher bzw. welchem man z. B. zur Mittelwertbildung der Signalintensitäten die einzelnen Werte in den Voxeln und/oder Pixeln heranzieht.

Gemäß der erfindungsgemäßen Technik können insbesondere über Segmentierungsmasken der Atlanten Untersuchungsbereiche und/oder ROIs ausgewählt werden. Alternativ oder ergänzend können die ROIs als Teil der Segmentierungsmasken und/oder identisch zu diesen betrachtet werden.

Der Tomograph kann einen medizinischen Scanner mit einer vorbestimmten Scan-Modalität (auch als Tomographie-Modalität und/oder Tomographen-Modalität bezeichnet) umfassen.

Die Scan-Modalität kann auch als bildgebendes Verfahren (insbesondere in der Medizin und insbesondere in der Radiologie) bezeichnet werden.

Die Tomographen-Modalität (auch als Scan-Modalität bezeichnet) kann eine Magnetresonanztomographie (MRT), Computertomographie (CT), und/oder CT-Angiographie umfassen.

In einer Erweiterung und/oder Verallgemeinerung der erfinderischen Technik können auch die Tomographen-Modalitäten Positronen-Emissions-Tomographie (PET), und/oder Einzelphotonen-Emissionscomputertomographie (fachsprachlich: Single Photon Emission Computed Tomography; kurz: SPECT), verwendet werden, wobei die "Unterabtastung" direkt über die Messzeit gesteuert wird (insbesondere, wie lange der Patient an einer Position gemessen wird). Alternativ oder ergänzend können PET und/oder SPECT eine (z. B. inhärent) stochastische Komponente umfassen, z. B. aufgrund von verwendetem Kontrastmittel, welches stochastisch pro Zeiteinheit und/oder Raumrichtung zerfällt.

Der synthetische Messdatensatz kann auch als multiparametrischer synthetischen anatomischer Datensatz bezeichnet werden. Beispielsweise kann der synthetische Messdatensatz jedes Atlas eine räumlichen Verteilung von (insbesondere synthetischen) physikalischen Parametern umfassen (in der MRT bspw. T1, T2, T2* Relaxationszeiten, Diffusionskonstanten, Wassergehalt und/oder Fettgehalt). Alternativ oder ergänzend kann der synthetische Messdatensatz eine Anatomie (insbesondere eines Patienten) darstellen.

Die Gruppe von Atlanten kann auch als Ensemble (und/oder Menge von Atlanten) bezeichnet werden. Die Gruppe von Atlanten kann Atlanten umfassen, die in mindestens einer Eigenschaft übereinstimmen, insbesondere hinsichtlich des (z. B. synthetischen) abgebildeten Bereichs des Körpers (z. B. Aufnahmen des Kopfbereichs) und/oder hinsichtlich einer möglichen (z. B. synthetischen) Pathologie, die mit der geplanten (insbesondere realen) medizinischen Aufnahme untersucht werden soll. Insbesondere durch die Beschränkung auf eine (z. B. synthetische) Pathologie können Rechenressourcen (z. B. Zeit, Prozessorkapazitäten und/oder Speicherkapazitäten) gespart werden beim Simulieren der Rekonstruktionen für jeden Atlas aus der ausgewählten Gruppe.

Die Bibliothek (fachsprachlich: Library) kann mehrere Gruppen von Atlanten umfassen. Beispielsweise können sich die Gruppen von Atlanten hinsichtlich der (z. B. synthetischen) abgebildeten Bereiche des Körpers unterscheiden. Insbesondere kann eine erste Gruppe Aufnahmen des Kopfbereichs umfassen und eine zweite Gruppe Aufnahmen des Rumpfbereichs. Alternativ oder ergänzend können sich die Gruppen von Atlanten hinsichtlich einer dargestellten (insbesondere synthetischen) Anomalie und/oder Läsion unterscheiden.

(Z. B. synthetische) Anatomien können in den Atlanten als Parameterbilder vorliegen (in der MRT z. B. als räumliche Verteilung von signalbeeinflussenden Parametern wie T1, T2, T2*, und/oder Diffusionskonstante). Die Parameterbilder können für eine Simulation des Messprozesses in Rohdatenräume (auch als k-Raum bezeichnet) verrechnet werden, insbesondere weil der k-Raum den Messprozess in der MRT beschreiben kann.

Alternativ oder ergänzend kann ein (z. B. synthetischer) Messdatensatz (insbesondere rohe, und/oder unverarbeitete) Messdaten umfassen, beispielsweise als Ergebnis der Simulation. Die (synthetischen und/oder realen) Messdaten können im Frequenzraum (auch als "k-Raum" bezeichnet) erfasst werden. Alternativ oder ergänzend können die synthetischen und/oder realen) Messdaten im Bildraum erfasst werden.

Der k-Raum ist ein Begriff, der für den Rohdatenraum von MRT-Messungen verwendet wird. Alternativ oder ergänzend können Rohdatenräume in der CT, PET, und/oder SPECT als Projektionen, Projektionsraum, und/oder Sinogramm bezeichnet werden.

Die Anfrage hinsichtlich der Planung der medizinischen Aufnahme kann eine Anatomie (z. B. für die die medizinische Aufnahme erstellt werden soll) umfassen.

Die (z. B. synthetische) Anatomie (auch als anatomische Struktur bezeichnet) kann ein Gelenk und/oder ein Band, das Gehirn, einen Tumor (und/oder eine anatomische Region umfassend einen Tumor), und/oder für Epilepsie charakteristische Bereiche umfassen.

Alternativ oder ergänzend kann die Anfrage hinsichtlich der Planung der medizinischen Aufnahme ferner einen Hinweis auf ein (z. B. bestehendes) Implantat umfassen. Beispielsweise kann ein Implantat zu einer Verzerrung eines Grundmagnetfelds (auch als B0-Verzerrung bezeichnet) eines MRT-Scans führen. Alternativ oder ergänzend kann ein Implantat zu einer Strahlaufhärtung bei einem CT-Scan führen.

Weiterhin alternativ oder ergänzend kann die Anfrage hinsichtlich der Planung der medizinischen Aufnahme mittels des Tomographen Informationen hinsichtlich des Tomographen, beispielsweise eine Leistungsfähigkeit des Tomographen, umfassen. Z. B. kann die Leistungsfähigkeit ein maximales (Gradienten-)Magnetfeld eines MRT-Scanners und/oder einen maximalen Röhrenstrom eines CT-Scanners umfassen.

Eine Simulation kann auch als Experiment "in silico" (und/oder am Computer) bezeichnet werden.

Die Simulation der ersten Rekonstruktion basierend auf dem vollständigen synthetischen Messdatensatz kann eine physikalische Simulation umfassen. Eine physikalische Simulation (beispielsweise in der Messschritte gemäß einem oder mehreren gegebenen Sequenzparametern auf eine Anatomie angewendet werden) kann in der MRT auf der Verwendung von Bloch-Gleichungen (oder Erweiterungen der Bloch-Gleichungen) basieren. Alternativ oder ergänzend kann die Simulation der ersten Rekonstruktion basierend auf dem vollständigen synthetischen Messdatensatz mittels einer trainierten KI ausgeführt werden.

Die Simulation der ersten Rekonstruktion kann als Ground Truth bezeichnet werden.

Die Simulation der mindestens einen zweiten Rekonstruktion basierend auf einem Anteil des synthetischen Messdatensatzes kann mittels einer trainierten KI ausgeführt werden. Die trainierte KI kann insbesondere ein tiefes neuronales Netzwerk (fachsprachlich: Deep Neural Network, DNN) umfassen. Das tiefe neuronale Netzwerk kann auch als tiefes künstliches neuronales Netzwerk bezeichnet werden. Ein neuronales Netzwerk kann auch kurz als neuronales Netz (NN) bezeichnet werden. Alternativ oder ergänzend kann die trainierte KI ein tiefes Lernen (fachsprachlich: Deep Learning, DL) umfassen.

Die mindestens eine zweite Rekonstruktion kann zwei oder mehr zweite Rekonstruktionen, beispielsweise mit unterschiedlichen auf dem Hinweis in der Anfrage basierenden Unterabtastungsfaktoren, (insbesondere pro Atlas) umfassen.

mindestens eine oder jede Rekonstruktion kann eine Rekonstruktion eines Bildes pro Unterspule und eine anschließende Rekonstruktion eines Gesamtbilds umfassen (beispielsweise, wenn eine Spule für einen MRT-Scan eine Mehrzahl von Unterspulen umfasst, beispielsweise zwischen 8 und 64 Unterspulen, insbesondere 8, 16, 32 oder 64 Unterspulen).

Die Qualitätsmetrik kann für die mindestens eine zweite Rekonstruktion eines Atlas bestimmt werden. Alternativ oder ergänzend kann die Qualitätsmetrik (beispielsweise als Mittelwert) für die ausgewählte Gruppe von Atlanten bestimmt werden.

Alternativ oder ergänzend kann die Qualitätsmetrik für die erste und/oder jede Rekonstruktion (z. B. individuell) eines Atlas bestimmt werden.

Die Qualitätsmetrik (auch: Qualitätsmaß, Qualitätsindex, Bildmetrik und/oder Güte) kann ein Kontrast-Rausch-Verhältnis (fachsprachlich: Contrast-to-Noise-Ratio, CNR), insbesondere bei Fettsättigung, und/oder ein Signal-Rausch-Verhältnis (auch: Störabstand oder Signal-Rauschabstand, kurz: Rauschabstand; fachsprachlich: Signal-to-Noise-Ratio, SNR), insbesondere ein Spitzen-Signal-Rausch-Verhältnis (fachsprachlich: Peak-SNR, SNR) umfassen. Alternativ oder ergänzend kann die Qualitätsmetrik einen Vergleich der CNRs und/oder SNRs der ersten Rekonstruktion und der mindestens einen zweiten Rekonstruktion umfassen. Weiterhin alternativ oder ergänzend kann die Qualitätsmetrik eine strukturelle Ähnlichkeit fachsprachlich: Structural Similarity; kurz: SSIM), eine mittlere quadratische Abweichung (auch: erwartete quadratische Abweichung, mittlerer quadratischer Fehler; fachsprachlich: Mean Squared Error, MSE), ein Differenzbild und/oder eine Transinformation (auch: gegenseitige Information; fachsprachlich: Mutual Information) der ersten Rekonstruktion und der mindestens einen zweiten Rekonstruktion umfassen (beispielsweise pro Atlas oder gemittelt über alle Atlanten der ausgewählten Gruppe von Atlanten).

Optional kann die Qualitätsmetrik eine Abschätzung einer Grö-ße einer Pathologie und/oder einer Läsion umfassen.

Das Bestimmen der Qualitätsmetrik kann für jede Rekonstruktion erfolgen. Beispielsweise können Einstellungen einer MRT-Bildgebungssequenz Grauwerte im Bild beeinflussen. Wird z. B. das CNR als Qualitätsmetrik verwendet, kann eine ROI im Areal der Läsion und eine ROI in einem gesunden Bereich ausgewertet werden (z. B. für die mindestens eine zweite Rekonstruktion und/oder mit Unterabtastung). Identischen Stellen und/oder ROIs können für die erste Rekonstruktion (und/oder ohne Unterabtastung, auch als voll abgetastetes Bild bezeichnet) ausgewertet werden und danach kann der Vergleich der erhaltenen Werte (z. B. zwischen der ersten Rekonstruktion und der mindestens einen zweiten Rekonstruktion) ausgeführt werden.

Das Ausgeben der bestimmten Qualitätsmetrik kann das Bereitstellen eines (insbesondere digitalen) Datensatzes umfassen. Das Ausgeben der bestimmten Qualitätsmetrik kann intern (beispielsweise in einer das Verfahren ausführenden Rechenvorrichtung und/oder in einem das Verfahren ausführenden System) erfolgen. Alternativ oder ergänzend kann die bestimmte Qualitätsmetrik auf einer Benutzeroberfläche (fachsprachlich: User Interface, UI), insbesondere einer graphischen Benutzeroberfläche (fachsprachlich: Graphical User Interface, GUI) ausgegeben werden.

Aufgrund der Ausgabe der bestimmten Qualitätsmetrik kann der Unterabtastungsfaktor für die geplante medizinische Aufnahme festgelegt und/oder die medizinische Aufnahme gestartet werden.

Die Schritte des Simulierens der ersten und mindestens einen zweiten Rekonstruktion, des Bestimmens einer Qualitätsmetrik und/oder des Ausgebens der Qualitätsmetrik können iterativ ausgeführt werden, beispielsweise je Atlas aus der ausgewählten Gruppe von Atlanten.

Das Verfahren kann als Remote Service, insbesondere entfernt von dem Tomographen (der auch als Scanner bezeichnet werden kann) für die geplante reale Messung, ausgeführt werden, beispielsweise an einer vom Tomographen räumlich getrennten (und/oder über eine drahtgebunden und/oder drahtlose Datenverbindung mit dem Tomographen verbundene) Rechenvorrichtung.

Die Tomographen-Modalität kann MRT umfassen. Alternativ oder ergänzend kann die Tomographen-Modalität CT und/oder CT-Angiographie umfassen.

In einer Erweiterung der erfindungsgemäßen Technik kann die Tomographen-Modalität ferner PET und/oder SPECT umfassen.

Mindestens ein oder jeder Atlas aus der Gruppe der Atlanten kann eine dem synthetischen Messdatensatz zugeordnete Annotation, insbesondere eine Segmentierungsmaske, umfassen.

Die Segmentierungsmaske kann mindestens ein Organ, eine anatomische Struktur und/oder eine Läsion umfassen. Alternativ oder ergänzend kann die Annotation eine Position einer Läsion (auch als krankhaftes Gewebe bezeichnet) und/oder eine Position von gesundem Gewebe umfassen.

Die Ground Truth kann die Simulation der ersten Rekonstruktion basierend auf dem vollständigen synthetischen Messdatensatz und/oder die Annotation, insbesondere die Segmentierungsmaske, umfassen.

Das Verfahren kann ferner einen Schritt des Ausgebens eines aufgrund der ausgegebenen Qualitätsmetrik festgelegten Unterabtastungsfaktors umfassen. Alternativ oder ergänzend kann das Verfahren ferner einen Schritt des Empfangens eines Verifikationssignal in Reaktion auf den, insbesondere ausgegebenen, festgelegten Unterabtastungsfaktor umfassen. Weiterhin alternativ oder ergänzend kann das Verfahren einen Schritt des Steuerns des Tomographen zur Ausführung der medizinischen Aufnahme mit dem festgelegten Unterabtastungsfaktor umfassen.

Die Ausgabe des aufgrund der ausgegebenen Qualitätsmetrik festgelegten Unterabtastungsfaktors kann eine Erhöhung und/oder eine Erniedrigung im Vergleich zum in der Anfrage enthaltenen Hinweis auf den Unterabtastungsfaktor umfassen.

Das Verifikationssignal kann über eine Benutzerschnittstelle (UI), insbesondere graphische Benutzeroberfläche (GUI), empfangen werden.

Das Steuern des Tomographen kann ein Startsignal umfassen.

Die Qualitätsmetrik kann ein Kontrast-Rausch-Verhältnis (CNR), insbesondere bei Fettsättigung, umfassen. Alternativ oder ergänzend kann die Qualitätsmetrik ein Signal-Rausch-Verhältnis (SNR) umfassen. Weiterhin alternativ oder ergänzend kann die Qualitätsmetrik eine Transinformation und/oder ein Differenzbild umfassen. Weiterhin alternativ oder ergänzend kann die Qualitätsmetrik ein mittlere quadratische Abweichung (MSE) umfassen. Weiterhin alternativ oder ergänzend kann die Qualitätsmetrik eine strukturelle Ähnlichkeit umfassen.

Der Vergleich der simulierten ersten Rekonstruktion und der simulierten mindestens einen zweiten Rekonstruktion kann einen Vergleich der gesamten Rekonstruktionen umfassen (beispielsweise je Atlas). Alternativ oder ergänzend kann der Vergleich der simulierten ersten Rekonstruktion und der simulierten mindestens einen zweiten Rekonstruktion einen Vergleich eines Bereichs von Interesse (ROI) umfassen (beispielsweise je Atlas). Der Bereich von Interesse kann in der Anfrage spezifiziert sein. Alternativ oder ergänzend kann der Bereich von Interesse auf der Annotation, insbesondere Segmentierungsmaske, basieren (beispielsweise je Atlas). Alternativ oder ergänzend kann der Vergleich basierend auf dem SNR und/oder CNR gesundes gegenüber krankem Gewebe pro Bild, oder nur krankes Gewebe umfassen. Weiterhin alternativ oder ergänzend kann für den Vergleich von Bildern eine Normierung, beispielsweise auf ein mittleres Signal des Bilds, vorgenommen werden.

Der synthetische Messdatensatz kann Messdaten in einem Frequenzraum umfassen. Eine Unterabtastung gemäß dem Unterabtastungsfaktor kann ein Überspringen von Frequenzen umfassen.

Der Frequenzraum kann auch als k-Raum bezeichnet werden. Die Rekonstruktion basierend auf dem Frequenzraum kann eine Schnelle Fourier-Transformation (fachsprachlich: Fast Fourier Transformation, FFT) umfassen. Alternativ oder ergänzend kann die Rekonstruktion basierend auf dem Frequenzraum eine Anwendung einer KI, insbesondere eines DNN, umfassen.

Das Überspringen von Frequenzen kann ungleichmäßig über den Frequenzraum verteilt sein. Beispielsweise können bevorzugt Frequenzen im Zentrum eines Frequenzbands abgetastet und Frequenzen an den Rändern des Frequenzbands übersprungen werden.

Alternativ oder ergänzend kann der synthetische Messdatensatz Messdaten in einem Bildraum umfassen. Eine Unterabtastung gemäß dem Unterabtastungsfaktor kann eine Wichtung und/oder ein Überspringen von Voxeln und/oder Pixeln und/oder Projektionen (z. B. bei einer CT-Aufnahme und/oder als CT-Spezifikum) umfassen.

Die Unterabtastung im Bildraum kann eine höhere Gewichtung von Voxeln und/oder Pixeln in einem Hauptbereich (z. B. im Zentrum und/oder einer oder mehrerer ROIs) des Bildbereichs und eine geringere Gewichtung an den Rändern des Bildbereichs umfassen. Alternativ oder ergänzend kann die Wichtung ein Überspringen von Voxeln und/oder Pixeln umfassen.

Der Unterabtastungsfaktor (und/oder Beschleunigungsfaktor) kann einen Wert größer als eins umfassen. Beispielsweise kann der Unterabtastungsfaktor im Wertebereich zwischen 2 und 100 liegen. Alternativ oder ergänzend kann ein Unterabtastungsfaktor mit Wert N einer Aufnahme jeder N-ten Zeile (und/oder N-ten Frequenz) entsprechen. Weiterhin alternativ oder ergänzend kann ein größerer Wert des Unterabtastungsfaktors einer schnelleren Messung (und/oder einer geringeren Anzahl an Messdaten) entsprechen.

Alternativ oder ergänzend kann der Unterabtastungsfaktor einen Prozentsatz (z. B. der aufnehmbaren Messdaten) umfassen. Beispielsweise kann ein Unterabtastungsfaktor von 50% oder 20% einer Aufnahme von aufnehmbaren Messdaten (z. B. als Zeilen und/oder Frequenzen) umfassen. Ein kleinerer Prozentsatz des Unterabtastungsfaktors kann einer schnelleren Messung (und/oder einer geringeren Anzahl an Messdaten) entsprechen.

Ein Unterabtastungsfaktor von 90% kann beispielsweise eine Aufnahme von 90% aller möglichen Messdaten (und/oder Messschritte) umfassen und/oder 10% aller möglichen Messdaten (und/oder Messschritte) auslassen. Der Unterabtastungsfaktor von 90% kann auch als Beschleunigungsfaktor von 1/0,9=1,11 als Wert größer 1 bezeichnet werden.

Ein Wert von 1 (und/oder 100%) des Unterabtastungsfaktors kann einer Aufnahme ohne Unterabtastung entsprechen.

Die Gruppe von Atlanten kann durch eine in jedem Atlas innerhalb der Gruppe erfasste anatomische Struktur und/oder Pathologie bestimmt sein.

Die Atlanten können sich hinsichtlich einer Größe, Art und/oder Lage einer Läsion bezüglich des Körpers eines Patienten unterscheiden.

Alternativ oder ergänzend können mindestens ein oder jeder Atlas einen Auflösungsmarker als Läsion umfassen, einen oder mehrere synthetische Messparameter gemäß einem Messprotokoll, ein Implantat, eine Kontrastmittelanflutung und/oder eine Bewegung (insbesondere eines Patienten) während der (z. B. simulierten) Messung.

(Z. B. synthetische) Messparameter gemäß einem Messprotokoll eines MRT-Scans können eine Anzahl Unterspulen und/oder eine Kontrastdimension umfassen. Alternativ oder ergänzend können (z. B. synthetische) Messparameter für einen MRT-Scan eine oder mehrere der Relaxationszeiten T1, T2 und/oder T2*, eine Diffusionskonstante, ein magnetische Suszeptibilität, ein Fett-Wasser-Verhältnis und/oder eine Metabiolitenkonzentration umfassen.

Die Anfrage hinsichtlich einer Planung einer medizinischen Aufnahme mittels eines Tomographen kann ferner einen Untersuchungsbereich und/oder einen Bereich von Interesse (ROI), einen (beispielsweise unteren und/oder minimalen) Schwellenwert der Qualitätsmetrik, eine Vorgabe einer Auflösung und/oder eine Vorgabe hinsichtlich eines oder mehrerer Messparameter umfassen.

Der Bereich von Interesse (ROI) kann einen räumlichen Teilbereich der medizinischen Aufnahme umfassen.

Der Schwellenwert der Qualitätsmetrik kann beispielsweise eine akustische Belastung (und/oder ein Rauschen) unterhalb (und/oder oberhalb) einer vorbestimmten Schwelle umfassen.

Der oder die Messparameter können eine (z. B. maximale) Messzeit und/oder einen Kontrast (z. B. basierend auf einer Relaxationszeit T1, T2 und/oder T2*) umfassen.

Das Bestimmen der Qualitätsmetrik kann eine Abschätzung einer Größe einer anatomischen Struktur und/oder einer Anomalie in der mindestens einen zweiten Rekonstruktion und/oder der ersten Rekonstruktion umfassen.

Beim Simulieren der mindestens zwei Rekonstruktionen kann ein Digitaler Zwilling (fachsprachlich: Digital Twin) des Tomographen verwendet werden, und/oder zum Einsatz kommen.

Durch die Verwendung des Digitaler Zwilling des Tomographen (auch: Scanners) können bei den Simulationen Prozessketten, Konfigurationen von Spulen (und/oder Unterspulen) und/oder Rauschquellen des Tomographen berücksichtigt werden. Alternativ oder ergänzend können für einen MRT-Scanner als Tomographen Verstärkerkennlinien und/oder Gradientenimperfektionen mitsimuliert werden.

Das Verfahren kann ferner einen Schritt des Durchführens einer Verifikationsmessung mittels des Tomographen umfassen.

Mittels der Verifikationsmessung kann (insbesondere nochmals) festgestellt werden, ob die Festlegung des Unterabtastungsfaktors für den medizinischen Zweck der medizinischen Aufnahme geeignet ist, beispielsweise hinsichtlich einer Auflösung.

Alternativ oder ergänzend kann das Verfahren ferner einen Schritt des Ausgebens, aufgrund der ausgegebenen Qualitätsmetrik, einer Einstellung und/oder Änderung der Einstellung mindestens eines, insbesondere physikalischen und/oder technischen, Parameters für die medizinische Aufnahme mittels des Tomographen umfassen.

Die physikalischen und/oder technischen Parameter für einen MRT-Scan und/oder ein MRT-Protokoll (auch: Sequenz) können eine Auflösung, eine oder mehrere Relaxationszeiten (T1, T2 und/oder T2*), eine Wichtung der Relaxationszeiten (z. B. T1w oder T2w), eine Repetitionszeit (TR), eine Echozeit (TE), eine Bandweite, eine Schichtdicke (fachsprachlich: slice thickness), ein Spulenprofil (z. B. mit Unterspulen und/oder Kanälen, insbesondere 8 bis 64 Kanäle für eine Kopfspule) und/oder ein Sichtfeld (fachsprachlich: Field of View; kurz: FoV) umfassen.

Alternativ oder ergänzend kann die Schichtdicke fachsprachlich eine "Slab thickness" umfassen, welche bei 3D-Aufnahmen einer "dicken" angeregten Schicht entspricht, welche mittels Phasenkodierung in dünne Schichten unterteilt und/oder "zerlegt" werden kann.

Alternativ oder ergänzend können die physikalischen und/oder technischen Parameter eine Messart umfassen, z. B. umfassend eine (z. B. einzige) Phasenkodierrichtung (fachsprachlich: single phase-encoding direction). Weiterhin alternativ oder ergänzend können die physikalischen und/oder technischen Parameter eine Messart, in der MRT bspw. Spin-Echoz. B., schnelles Spin-Echo (auch als rapid acquisition with relaxation enhancement, RARE, bezeichnet, und/oder als Turbo-Spin-Echo und/oder Fast Spin-Echo), schnelles Spin-Echo T1-gewichtet, schnelles Spin-Echo T2-gewichtet und/oder T2 fluid-attenuated inversion recovery (FLAIR) umfassen. Fluidattenuated kann auch als "Flüssigkeitsunterdrückung" und/oder "flüssigkeitsunterdrückt" bezeichnet werden. Die FLAIR Technik kann eine RARE-Sequenz mit speziellen Einstellungen und einem zusätzlichen Hochfrequenzpuls umfassen. Alternativ oder ergänzend kann FLAIR auf deutsch als T2-gewichtete flüssigkeitsunterdrückte RARE-Bildgebung bezeichnet werden.

Die physikalischen und/oder technischen Parameter für einen CT-Scan und/oder CT-Protokolle können einen Röhrenstrom, eine Rotationsgeschwindigkeit und/oder einen Rekonstruktionskernel umfassen.

Die physikalischen und/oder technischen Parameter (beispielsweise ca. 80 Parameter für einen MRT-Scan) können im Allgemeinen Interdependenzen haben bzw. nicht alle voneinander unabhängig sein.

Gemäß einem Vorrichtungsaspekt ist eine Rechenvorrichtung zur Bestimmung eines Unterabtastungsfaktors für eine medizinische Aufnahme mittels eines Tomographen bereitgestellt. Die Rechenvorrichtung umfasst eine Eingabeschnittstelle, die dazu ausgebildet ist, eine Anfrage hinsichtlich einer Planung einer medizinischen Aufnahme mittels eines Tomographen zu empfangen. Die Anfrage umfasst einen Hinweis auf einen Unterabtastungsfaktor. Die Rechenvorrichtung umfasst ferner ein Auswahlmodul, das dazu ausgebildet ist, eine Gruppe von Atlanten aus einer Bibliothek auszuwählen. Jeder Atlas in der Bibliothek umfasst einen synthetischen Messdatensatz zu einer vorbestimmten Tomographen-Modalität (z. B. MRT). Das Auswählen der Gruppe von Atlanten basiert auf der empfangenen Anfrage hinsichtlich der Planung der medizinischen Aufnahme mittels des Tomographen (z. B. im Hinblick auf eine aufzunehmende anatomische Struktur).

Die Rechenvorrichtung umfasst ferner ein Simulationsmodul (oder eine Schnittstelle zu einem, insbesondere externen, Simulationsmodul, das als KI ausgebildet sein kann), das dazu ausgebildet ist, für jeden Atlas aus der ausgewählten Gruppe von Atlanten mindestens zwei Rekonstruktionen zu simulieren zum Erstellen jeweils eines Bilddatensatzes. Eine erste Rekonstruktion basiert auf dem vollständigen synthetischen Messdatensatz. Mindestens eine zweite Rekonstruktion basiert auf einem Anteil des synthetischen Messdatensatzes entsprechend des mit der Anfrage empfangenen Hinweises auf den Unterabtastungsfaktor.

Die Rechenvorrichtung umfasst ferner ein Bestimmungsmodul, das dazu ausgebildet ist, eine Qualitätsmetrik der mindestens einen zweiten Rekonstruktion zu bestimmen (insbesondere für jeden Atlas aus der ausgewählten Gruppe von Atlanten, und/oder als Mittelwert derselben). Alternativ oder ergänzend ist das Bestimmungsmodul dazu ausgebildet, für die erste und/oder jede weitere Rekonstruktion eine Qualitätsmetrik zu bestimmen.

Das Bestimmen der Qualitätsmetrik umfasst ein Vergleichen der simulierten mindestens einen zweiten Rekonstruktion mit der simulierten ersten Rekonstruktion (insbesondere für jeden Atlas aus der ausgewählten Gruppe von Atlanten, und/oder als Mittelwert derselben). Die Rechenvorrichtung umfasst ferner eine Ausgabeschnittstelle, die dazu ausgebildet ist, die bestimmte Qualitätsmetrik zum Festlegen des Unterabtastungsfaktors für die medizinische Aufnahme auszugeben.

Die Rechenvorrichtung kann ferner dazu ausgebildet sein, einen beliebigen oder jeden Schritt des Verfahrens gemäß dem Verfahrensaspekt auszuführen. Alternativ oder ergänzend kann die Rechenvorrichtung ferner dazu ausgebildet sein, im Zusammenhang mit dem Verfahren gemäß dem Verfahrensaspekt offenbarte Merkmale zu umfassen.

Gemäß einem Systemaspekt ist ein System zur Bestimmung eines Unterabtastungsfaktors für eine medizinische Aufnahme mittels eines Tomographen bereitgestellt. Das System umfasst eine Rechenvorrichtung gemäß dem Vorrichtungsaspekt und einen Tomographen, mittels dem eine medizinische Aufnahme geplant wird.

In dem Fall, dass die Rechenvorrichtung eine Schnittstelle zu einem (insbesondere externen) Simulationsmodul, das als KI ausgebildet sein kann, umfasst, kann das System ferner das Simulationsmodul (und/oder die KI) umfassen.

Gemäß einem weiteren Aspekt ist ein Computerprogrammprodukt bereitgestellt mit Programmelementen, die eine Rechenvorrichtung veranlassen, die Schritte des Verfahrens zur Bestimmung eines Unterabtastungsfaktors für eine medizinische Aufnahme mittels eines Tomographen gemäß dem Verfahrensaspekt auszuführen, wenn die Programmelemente in einen Speicher der Rechenvorrichtung geladen werden.

Gemäß einem weiteren Aspekt ist ein computerlesbares Speichermedium bereitgestellt, auf dem Programmelemente gespeichert sind, die von einer Rechenvorrichtung gelesen und ausgeführt werden können, um Schritte des Verfahrens zur Bestimmung eines Unterabtastungsfaktors für eine medizinische Aufnahme mittels eines Tomographen gemäß dem Verfahrensaspekt durchzuführen, wenn die Programmelemente von der Rechenvorrichtung ausgeführt werden.

Die oben beschriebenen Eigenschaften, Merkmale und Vorteile der vorliegenden Erfindung sowie die Art und Weise, wie sie erreicht werden, werden im Lichte der folgenden Beschreibung und der Ausführungsbeispiele, die im Zusammenhang mit den Zeichnungen näher erläutert werden, klarer und verständlicher. Diese folgende Beschreibung beschränkt die Erfindung nicht auf die enthaltenen Ausführungsformen. Gleiche Komponenten oder Teile können in verschiedenen Figuren mit den gleichen Bezugszeichen versehen sein. Im Allgemeinen sind die Abbildungen nicht maßstabsgetreu.

Es versteht sich, dass eine bevorzugte Ausführungsform der vorliegenden Erfindung auch eine beliebige Kombination der abhängigen Ansprüche oder der obigen Ausführungsformen mit dem jeweiligen unabhängigen Anspruch sein kann.

Diese und andere Aspekte der Erfindung werden aus den nachfolgend beschriebenen Ausführungsformen ersichtlich und werden durch Bezugnahme auf diese erläutert.

### KURZE BESCHREIBUNG DER ZEICHNUNGEN

- Fig. 1: ist ein Flussdiagramm eines Verfahrens zur Bestimmung eines Unterabtastungsfaktors für eine medizinische Aufnahme mittels eines Tomographen gemäß einer bevorzugten Ausführungsform der vorliegenden Erfindung;
- Fig. 2: ist eine Übersicht über die Struktur und den Aufbau einer Rechenvorrichtung zur Bestimmung eines Unterabtastungsfaktors für eine medizinische Aufnahme mittels eines Tomographen gemäß einer bevorzugten Ausführungsform der vorliegenden Erfindung, wobei die Rechenvorrichtung dazu ausgebildet sein kann, das Verfahren der Fig. 1 auszuführen;
- Figs. 3A und 3B: zeigen ein weiteres beispielhaftes Flussdiagramm des Verfahrens zur Bestimmung eines Unterabtastungsfaktors für eine medizinische Aufnahme mittels eines Tomographen, das eine Variante des Verfahrens der Fig. 1 sein kann; und
- Figs. 4A, 4B und 4C: zeigen Bildschirmansichten für die Wahl eines beispielhaften physikalischen und/oder technischen Parameters für eine medizinische Aufnahme, der die Änderung eines weiteren beispielhaften physikalischen und/oder technischen Parameters erfordert, um eine vorbestimmte Qualität (z. B. parametrisiert mittels einer Qualitätsmetrik) der medizinischen Aufnahme zu gewährleisten.

Etwaige Bezugszeichen in den Ansprüchen sind nicht als Einschränkung des Anwendungsbereichs zu verstehen.

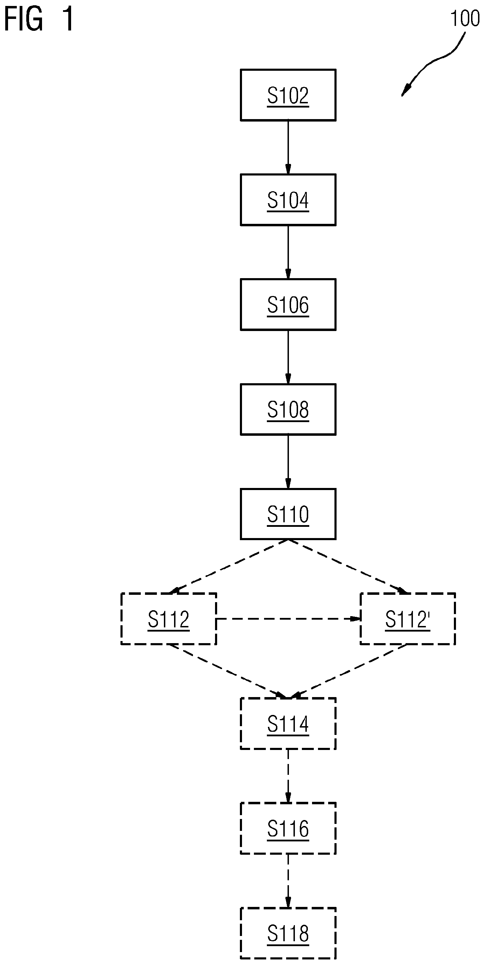

Fig. 1 zeigt schematisch ein (insbesondere computer-implementiertes) Verfahren zur Bestimmung eines Unterabtastungsfaktors für eine medizinische Aufnahme mittels eines Tomographen. Das Verfahren ist allgemein mit Bezugszeichen 100 bezeichnet.

Das Verfahren 100 umfasst einen Schritt S102 des Empfangens einer Anfrage hinsichtlich einer Planung einer medizinischen Aufnahme mittels eines Tomographen. Die Anfrage umfasst einen Hinweis auf einen Unterabtastungsfaktor.

Das Verfahren 100 umfasst ferner einen Schritt S104 des Auswählens einer Gruppe von Atlanten aus einer Bibliothek. Jeder Atlas in der Bibliothek umfasst einen synthetischen Messdatensatz zu einer vorbestimmten Tomographen-Modalität (z. B. MRT). Das Auswählen S104 der Gruppe von Atlanten basiert auf der empfangenen S102 Anfrage hinsichtlich der Planung der medizinischen Aufnahme mittels des Tomographen (z. B. hinsichtlich einer aufzunehmenden anatomischen Struktur).

Das Verfahren 100 umfasst ferner einen Schritt S106 des Simulierens von mindestens zwei Rekonstruktionen zum Erstellen jeweils eines Bilddatensatzes für jeden Atlas aus der ausgewählten S104 Gruppe von Atlanten. Eine erste Rekonstruktion basiert auf dem vollständigen synthetischen Messdatensatz. Mindestens eine zweite Rekonstruktion basiert auf einem Anteil des synthetischen Messdatensatzes entsprechend des mit der Anfrage empfangenen S102 Hinweises auf den Unterabtastungsfaktor.

Das Verfahren 100 umfasst ferner einen Schritt S108 des Bestimmens einer Qualitätsmetrik der mindestens einen zweiten Rekonstruktion (insbesondere für jeden Atlas aus der ausgewählten S104 Gruppe von Atlanten, und/oder als Mittelwert derselben). Das Bestimmen S108 der Qualitätsmetrik umfasst ein Vergleichen der simulierten S106 mindestens einen zweiten Rekonstruktion mit der simulierten S106 ersten Rekonstruktion (insbesondere je Atlas aus der ausgewählten S104 Gruppe von Atlanten).

Das Verfahren 100 umfasst ferner einen Schritt S110 des Ausgebens der bestimmten S108 Qualitätsmetrik zum Festlegen des Unterabtastungsfaktors für die medizinische Aufnahme.

Optional umfasst das Verfahren 100 einen Schritt S112 des Ausgebens S112 eines aufgrund der ausgegebenen S110 Qualitätsmetrik festgelegten Unterabtastungsfaktors.

Alternativ oder ergänzend kann das Verfahren einen Schritt S112'des Ausgeben, aufgrund der ausgegebenen S110 Qualitätsmetrik, einer Einstellung und/oder Änderung der Einstellung mindestens eines, insbesondere physikalischen und/oder technischen, Parameters für die medizinische Aufnahme mittels des Tomographen umfassen.

Weiterhin optional umfasst das Verfahren einen Schritt S114 des Empfangens eines Verifikationssignal in Reaktion auf den, insbesondere ausgegebenen S112, festgelegten Unterabtastungsfaktor.

Weiterhin optional umfasst das Verfahren einen Schritt S116 des Durchführens einer Verifikationsmessung mittels des Tomographen.

Weiterhin optional umfasst das Verfahren einen Schritt S118 des Steuerns des Tomographen zur Ausführung der medizinischen Aufnahme mit dem festgelegten Unterabtastungsfaktor.

Die Schritte S106 des Simulierens der mindestens zwei Rekonstruktionen je Atlas, des Bestimmens S108 der zugehörigen Qualitätsmetrik und/oder des Ausgebens S110 der bestimmten S108 Qualitätsmetrik können iterativ ausgeführt werden, jeweils einzeln für jeden Atlas.

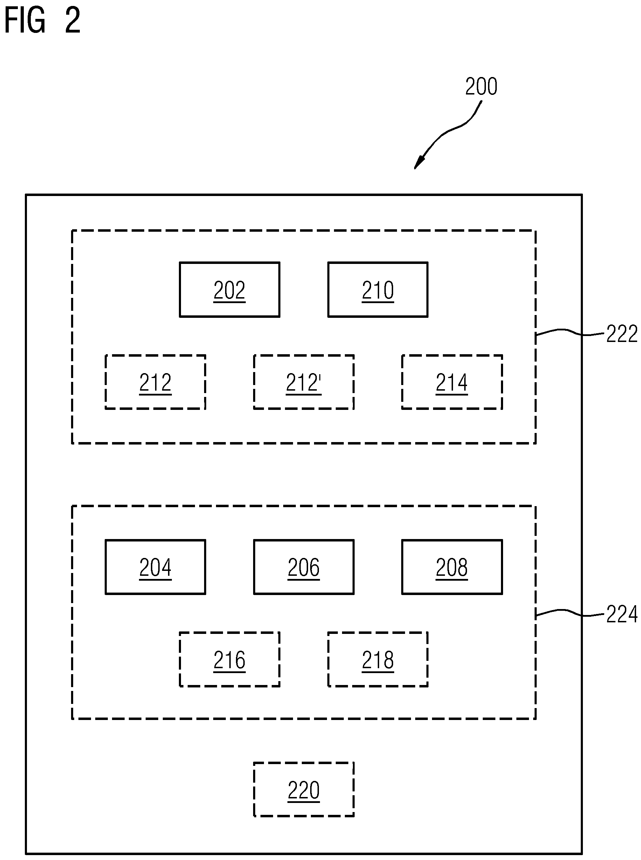

Fig. 2 zeigt schematisch eine Rechenvorrichtung zur Bestimmung eines Unterabtastungsfaktors für eine medizinische Aufnahme mittels eines Tomographen. Die Rechenvorrichtung ist allgemein mit dem Bezugszeichen 200 bezeichnet.

Die Rechenvorrichtung 200 umfasst eine Eingabeschnittstelle 202, die dazu ausgebildet ist, eine Anfrage hinsichtlich einer Planung einer medizinischen Aufnahme mittels eines Tomographen zu empfangen. Die Anfrage umfasst einen Hinweis auf einen Unterabtastungsfaktor.

Die Rechenvorrichtung 200 umfasst ferner ein Auswahlmodul 204, das dazu ausgebildet ist, eine Gruppe von Atlanten aus einer Bibliothek auszuwählen. Jeder Atlas in der Bibliothek umfasst einen synthetischen Messdatensatz zu einer vorbestimmten Tomographen-Modalität (z. B. MRT). Das Auswählen der Gruppe von Atlanten basiert auf der empfangenen Anfrage hinsichtlich der Planung der medizinischen Aufnahme mittels des Tomographen (z. B. hinsichtlich einer aufzunehmenden anatomischen Struktur).

Die Rechenvorrichtung 200 umfasst ferner ein Simulationsmodul 206 oder eine Schnittstelle zu einem Simulationsmodul 206. Das Simulationsmodul 206 ist dazu ausgebildet, für jeden Atlas aus der ausgewählten Gruppe von Atlanten mindestens zwei Rekonstruktionen zu simulieren zum Erstellen jeweils eines Bilddatensatzes. Eine erste Rekonstruktion basiert auf dem vollständigen synthetischen Messdatensatz. Mindestens eine zweite Rekonstruktion basiert auf einem Anteil des synthetischen Messdatensatzes entsprechend des mit der Anfrage empfangenen Hinweises auf den Unterabtastungsfaktors.

Die Rechenvorrichtung 200 umfasst ferner ein Bestimmungsmodul 208, das dazu ausgebildet ist, eine Qualitätsmetrik der mindestens einen zweiten Rekonstruktion zu bestimmen (insbesondere für jeden Atlas aus der ausgewählten Gruppe von Atlanten, und/oder als Mittelwert derselben). Das Bestimmen der Qualitätsmetrik umfasst ein Vergleichen der simulierten mindestens einen zweiten Rekonstruktion mit der simulierten ersten Rekonstruktion (insbesondere je Atlas aus der ausgewählten Gruppe von Atlanten).

Die Rechenvorrichtung 200 umfasst ferner eine (insbesondere erste) Ausgabeschnittstelle 210, die dazu ausgebildet ist, die bestimmte Qualitätsmetrik zum Festlegen des Unterabtastungsfaktors für die medizinische Aufnahme auszugeben.

Optional umfasst die Rechenvorrichtung 200 eine zweite Ausgabeschnittstelle 212, die dazu ausgebildet ist, einen aufgrund der ausgegebenen Qualitätsmetrik festgelegten Unterabtastungsfaktor auszugeben.

Alternativ oder ergänzend kann die Rechenvorrichtung 200 eine dritte Ausgabeschnittstelle 212' umfassen, die dazu ausgebildet ist, aufgrund der ausgegebenen Qualitätsmetrik eine Einstellung und/oder Änderung der Einstellung mindestens eines, insbesondere physikalischen und/oder technischen, Parameters für die medizinische Aufnahme mittels des Tomographen auszugeben.

Optional umfasst die Rechenvorrichtung 200 ferner eine Empfangsschnittstelle 214, die dazu ausgebildet ist, eine Verifikationssignal in Reaktion auf den, insbesondere ausgegebenen, festgelegten Unterabtastungsfaktor zu empfangen.

Alternativ oder ergänzend kann die Rechenvorrichtung 200 ein Verifikationsmessungsmodul 216 umfassen, das dazu ausgebildet ist, eine Verifikationsmessung mittels des Tomographen durchzuführen.

Weiterhin alternativ oder ergänzend kann die Rechenvorrichtung 200 ein Steuermodul 218 umfassen, das dazu ausgebildet ist, den Tomographen zur Ausführung der medizinischen Aufnahme mit dem festgelegten Unterabtastungsfaktor zu steuern.

Ferner kann die Rechenvorrichtung 200 einen Speicher 220 umfassen, beispielsweise zur Speicherung eines Computerprogramms zur Durchführung des Verfahrens 100.

Die Eingabeschnittstelle 202, die (insbesondere erste) Ausgabeschnittstelle 210, die optionale zweite Ausgabeschnittstelle 212, die optionale dritte Ausgabeschnittstelle 212', und/oder die optionale Empfangsschnittstelle 214 können in einer Eingabe-Ausgabe-Schnittstelle 222 der Rechenvorrichtung 200 umfasst sein. Alternativ oder ergänzend kann die Schnittstelle zu dem Simulationsmodul 206 in der Eingabe-Ausgabe-Schnittstelle 222 der Rechenvorrichtung 200 umfasst sein.

Das Auswahlmodul 204, das Simulationsmodul 206, das Bestimmungsmodul 208, das optionale Steuermodul 216, und/oder das optionale Verifikationsmessungsmodul 218 können in einem Prozessor 224 der Rechenvorrichtung 200 umfasst sein.

Die Rechenvorrichtung 200 kann dazu ausgebildet sein, das Verfahren 100 auszuführen.

Ein System kann die Rechenvorrichtung 200, einen Tomographen, mittels dem die geplante medizinische Aufnahme aufgenommen werden soll, und optional eine KI (beispielsweise als Simulationsmodul 206, insbesondere wenn die Rechenvorrichtung 200 nur eine Schnittstelle zu dem Simulationsmodul 206 umfasst) umfassen.

Die erfinderische Technik (z. B. umfassend das Verfahren 100, die Rechenvorrichtung 200, und/oder das System) kann die Rekonstruktion anatomisch ähnlicher Bilddaten (z. B. mit bekannter Ground Truth und/oder Annotation) ermöglichen.

Die erfinderische Technik kann auch als simulationsbasierte Qualitätskontrolle bei mehrschichtigen Lernen (auch: tiefes Lernen oder tiefgehendes Lernen; fachsprachlich: Deep Learning, DL)-Rekonstruktionstechniken bezeichnet werden.

In der Rechenvorrichtung 200 (die auch als Bildrekonstruktionsrechner bezeichnet werden kann) kann eine geeignete Bibliothek an Atlanten (und/oder synthetischen Anatomien, auch als synthetische Daten bezeichnet) mit unterschiedlichen, bekannten und segmentierten Läsionen unterschiedlicher Größe und Art hinterlegt sein (z. B. im Speicher 220), z. B. mindestens zehn Kopfdaten mit diffusen Tumoren und/oder mindestens zehn Kopfdaten mit AVMs.

Die MRT-Messung kann mit den Daten des Protokolls an diesen synthetischen Messdaten (kurz auch: synthetischen Daten) simuliert werden (insbesondere im Schritt S106). Zusätzlich kann die MRT-Messung ohne Beschleunigung simuliert werden (z. B. im Schritt S106). In den so erzeugten synthetischen MRT-Daten kann durch geeignete Vergleiche (z. B. CNR-Vergleich, Mutual Information zwischen MRT-Bildern mit und/oder ohne Beschleunigung und/oder Ground Truth, und/oder Differenzbilder) abgeschätzt werden, ob die Läsionen noch sichtbar sind (z. B. im Schritt S108 des Bestimmens der Qualitätsmetrik).

Gemäß einem Ausführungsbeispiel, das mit jedem weiteren Ausführungsbeispiel kombinierbar ist, kann eine Art Auflösungsmarker als Läsion (z. B. in die synthetischen Daten) eingebracht werden, so dass auch die gerade noch rekonstruierte Größe abschätzbar wird.

Die Information (z. B. Zahl der noch korrekt dargestellten Läsionen in der Bibliothek) kann gemäß einem Ausführungsbeispiel, das mit jedem anderen Ausführungsbeispiel kombinierbar ist, einem Nutzer (auch Benutzer und/oder Anwender) vor der Aufnahme (die auch als Messung bezeichnet werden kann) mitgeteilt werden, so dass wieder eine Intuition (insbesondere beim Nutzer) für den Wahrheitsgehalt der realen (auch; echten) MRT-Bilder entsteht.

Der erfinderischen Technik liegt die Überlegung zugrunde, dass sich die ausgewählte Gruppe von Atlanten (und/oder das Ensemble der synthetischen Daten bezeichnet) ähnlich verhalten wird, wie Läsionen des Patienten, deren (z. B. genaue) Position und Art vorab unbekannt sind.

Die erfinderische Technik ermöglicht eine flexiblere Protokollgestaltung auf Seiten des Tomographen (z. B. MRT)-Anwenders durch freiere Parameterwahl. Des Weiteren können, insbesondere im Simulationsschritt S106 verwendete, DL-Modelle ausgetauscht werden (z. B. durch Upgrades) und die dem Nutzer zur Verfügung stehenden Grenzen können sich entsprechend verschieben.

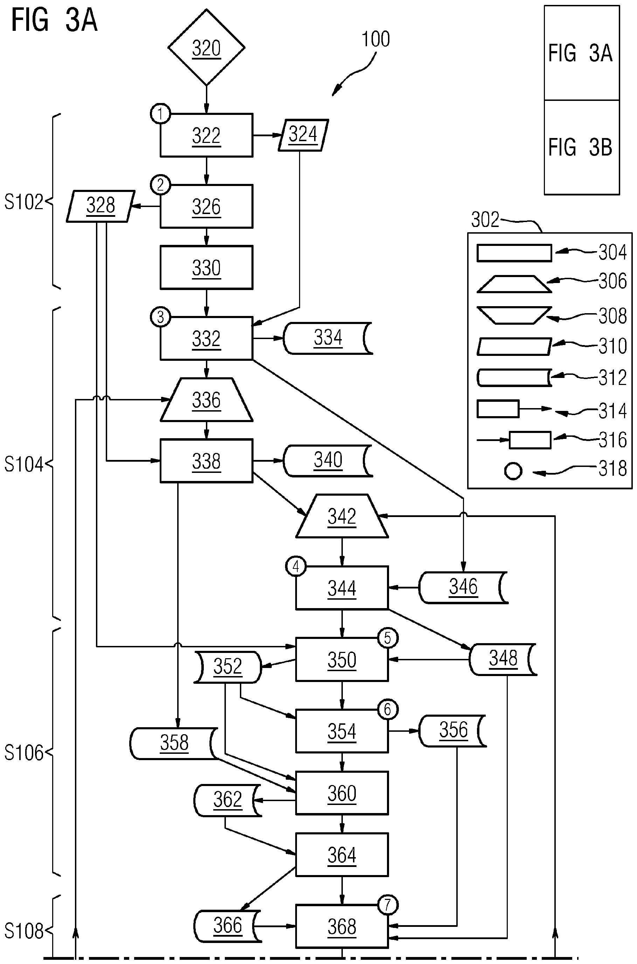

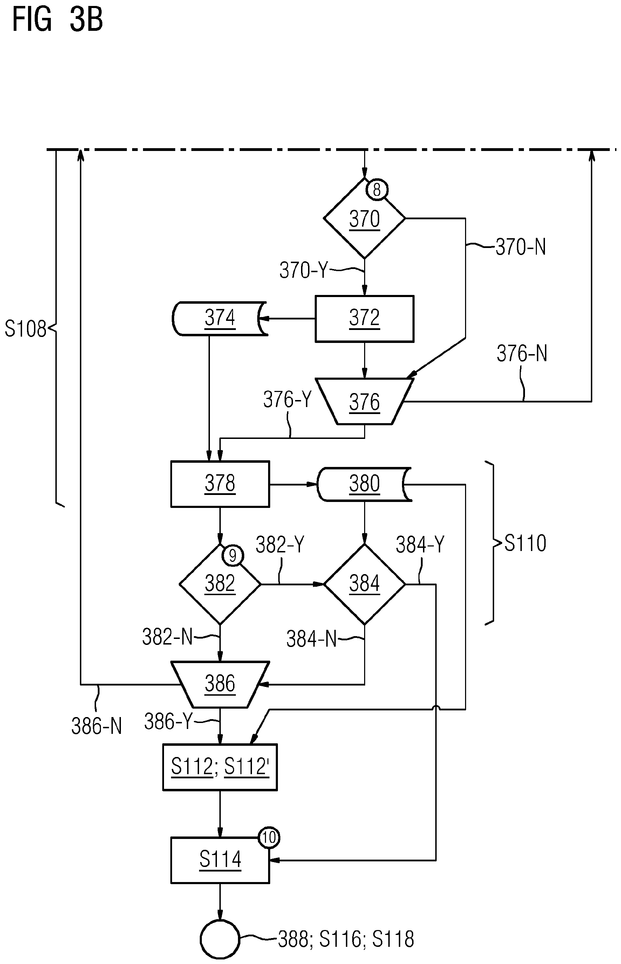

Figs. 3A und 3B zeigen ein Ausführungsbeispiel des erfindungsgemäßen Verfahrens 100 zur Bestimmung eines Unterabtastungsfaktors für eine medizinische Aufnahme mittels eines Tomographen (das auch als Simulationsbasierte Qualitätskontrolle bei DL-Rekonstruktionstechniken bezeichnet werden kann).

In dem mit Bezugszeichen 302 bezeichneten Kasten ist eine Legende der verschiedenen Formen des Flussdiagramms in Figs. 3A und 3B wie folgt gegen: Rechtecke gemäß Bezugszeichen 304 bezeichnen Aktionsschritte (und/oder Verfahrensschritte). Sich nach oben verjüngende Trapeze 306 beschreiben jeweils einen Schleifenanfang. Sich nach unten verjüngende Trapeze 308 beschreiben jeweils ein Schleifenende. Ein Parallelogramm 310 beschreibt Metadaten. Ein nach links abgerundetes Rechteck 312 beschreibt Rechendaten. Ein aus einem Rechteck austretender Pfeil, wie an Bezugszeichen 314 gezeigt, beschreibt das Ausgeben von Daten aus einem Aktionsschritt (und/oder Verfahrensschritt). Ein in ein Rechteck mündender Pfeil, wie an Bezugszeichen 316 gezeigt, beschreibt den Erhalt von Daten für einen Aktionsschritt und/oder Verfahrensschritt). Mit einem Kreis 318 sind zehn im folgenden Begleittext näher beschriebene Schritte des beispielhaften Verfahrens 100 der Figs. 3A und 3B bezeichnet.

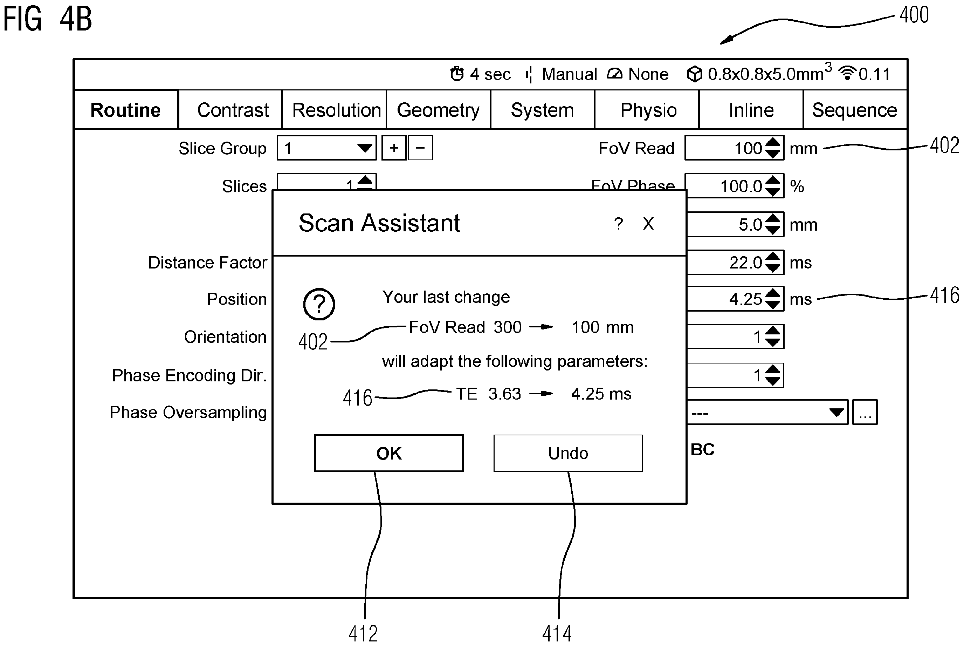

Zur näheren Beschreibung der Figs. 3A und 4B werden folgende Bezeichnungen und Definitionen verwendet.

K-Raum-Daten umfassen simulierte und/oder gemessene Rohdaten einer MRT-Messung (auch als MRT-Scan bezeichnet). Die simulierten und/oder gemessenen Rohdaten liegen im (z. B. Orts)-Frequenzraum vor. Beispielsweise ohne Unterabtastung können k-Raumdaten konventionellerweise über eine FFT zu Bildern umgerechnet werden. Wird eine Spule mit mehreren Kanälen (und/oder Unterspulen) für die MRT-Messung verwendet, so hat der resultierende k-Raum eine zusätzliche Dimension in welcher die individuellen Messdaten der einzelnen Unterspulen (und/oder Kanäle) gespeichert sind.

Im Rahmen der erfinderischen Technik umfassen synthetische Daten künstlich erzeugte Datensätze, welche eine anatomische Region (auch als anatomische Struktur, Anatomie und/oder ROI bezeichnet) darstellen (und/oder abbilden). Die synthetischen Daten liegen möglichst hochaufgelöst vor und bestehen neben den drei Ortsdimensionen noch aus einer zusätzlichen Kontrastdimension, in der MRT-relevante (insbesondere physikalische und/oder technische) Parameter vorhanden sind (z. B. T1, T2, T2* und/oder Diffusionskonstante). Es ist möglich, dass für unterschiedliche MRT-Sequenzen nur ein Teil der (insbesondere physikalischen und/oder technischen) Parameter für die Simulation relevant sind. Alternativ oder ergänzend kann es möglich sein, dass weitere insbesondere physikalischen und/oder technischen) Parameter benötigt werden (z. B. magnetische Suszeptibilität, Fett-Wasser-Verhältnis und/oder Metabiolitenkonzentrationen). Synthetische Daten können neben Daten für gesundes Gewebe auch typische Daten für krankhafte Veränderungen (z. B. Tumore) beinhalten. Das krankhafte Gewebe kann an unterschiedlichen Stellen in den synthetischen Daten in unterschiedlichen Größen dargestellt sein.

Im Rahmen der erfinderischen Technik umfasst ein Atlas einen Satz aus synthetischen Daten und optional zugehörigen Segmentierungsmasken, z. B. Positionsangaben, welches Gewebe als gesund und welches als krankhaft angesehen werden kann. Über die Segmentierungsmaske kann im Schritt S108 des Bestimmens der Qualitätsmetrik (auch als Algorithmenschritt bezeichnet) ein Vergleich zwischen unbeschleunigtem Bild (das auch als Ground Truth bezeichnet werden kann) und beschleunigtem Bild gezogen werden, indem z. B. Signalunterschiede und/oder Kontrastunterschiede zwischen gesundem und krankem Gewebe in den jeweiligen Bildern bestimmt (z. B. berechnet) und zwischen beschleunigter und unbeschleunigter Aufnahme (insbesondere computergestützt) vergleichen werden können.

Im Rahmen der erfinderischen Technik umfasst ein Ensemble (auch als ausgewählte Gruppe von Atlanten bezeichnet) eine Zusammenstellung mehrerer Atlanten, welche beispielsweise zu einer Pathologie gehören. Die einzelnen Atlanten des Ensembles (und/oder der ausgewählten Gruppe) können z. B. unterschiedliche Läsionen (z. B. unterschiedliche Arten, Größen und/oder Positionen von Tumoren) umfassen und können (und/oder sollen) die klinische Variabilität der Pathologie darstellen.

Das Ensemble (und/oder die ausgewählte Gruppe) sollte (bzw. muss) groß genug sein, um genügend typische Fälle (z. B. von Läsionen und/oder Pathologien) abzubilden. Eine Grundidee der erfinderischen Technik ist, dass die Beschleunigung einer (z. B. MRT-) Messung so gewählt werden kann, dass die in den Atlanten abgebildeten Pathologien darstellbar bleiben, wodurch die Annahme gerechtfertigt ist, dass auch ein (insbesondere vermutete und/oder zu untersuchende) Läsion der oder des typischen klinischen Patientin oder Patienten darstellbar (und/oder abbildbar und/oder aufnehmbar) sein wird.

Im Rahmen der erfinderischen Technik umfasst eine Ground Truth einen aus einem synthetischen Datensatz simulierten k-Raum, welcher frei von Unterabtastung ist und z. B. mittels konventioneller FFT rekonstruiert werden kann. Die Simulation des k-Raums kann dieselben MRT-Parameter annehmen, welche der Nutzer gesetzt hat. Dies dient dem Zweck, dass die Ground Truth und das beschleunigte Bild dieselbe Kontrastverteilung bzw. dieselbe Bildwichtung erfahren können.

Im Rahmen der erfinderischen Technik umfasst eine (beispielsweise iterative) Rekonstruktion ein Verfahren (und/oder ein Ergebnis des Verfahrens), das aus einem Rohdatenraum und/oder einem k-Raum einen Bilddatensatz erzeugt.

Eine iterative Rekonstruktion kann ein Sammelbegriff für (z. B. viele) unterschiedliche Algorithmen sein (beispielsweise anstelle einer Bezeichnung eines Algorithmus). Den (z. B. vielen) unterschiedlichen Algorithmen kann gemein sein, dass eine Lösung (und/oder ein Ergebnis) schrittweise (und/oder iterativ) durch wiederholte Auswertung einer Funktion geschieht (und/oder erhalten wird), insbesondere in welcher ein Feedback der vorherigen Rekonstruktionsschritte stattfindet und so die Lösung schrittweise immer weiter verbessert wird.

Dies kann konventionell per FFT und/oder über KI geschehen , wobei in allen Fällen auch weitere Verarbeitungsschritte nach dem Stand der Technik verwendet werden können (z. B. klinisch etablierte Techniken der parallelen Bildgebung wie GRAPPA, SENSE und/oder partial-Fourier-Rekonstruktion).

Die FFT kann eine Laufzeitkomplexität von O(N*log(N)) für eine Eingabedatengröße N umfassen. Alternativ oder ergänzend kann eine konventionelle Diskrete Fourier-Transformation (DFT) eine Laufzeitkomplexität von O(N^2)_umfassen.

Die FFT kann einen vollständig abgetasteten Datensatz (und/oder vollständigen k-Raum) benötigen, um artefakt-freie Bilder zu rekonstruieren. Alternativ oder ergänzend können bei Anwendung der FFT bei fehlenden (und/oder unterabgetasteten) Daten Artefakte entstehen.

Werden z. B. Mehrkanalspulen verwendet, kann als Zwischenschritt pro Unterspule ein separater Bilddatensatz entstehen. Die Daten des separaten Bilddatensatzes pro Unterspule können miteinander kombiniert (und/oder verrechnet) werden, so dass aus den einzelnen Bildern der Unterspulen ein einziger Bilddatensatz entsteht.

Das Ausführungsbeispiel des Verfahrens 100 für die Bestimmung eines Unterabtastungsfaktors für eine medizinische MRT-Aufnahme in Fig. 3A beginnt (und/oder startet) an Bezugszeichen 320.

An Bezugszeichen 322 wählt der Nutzer eine oder mehrere Spulen und eine zu untersuchende Art der Pathologie (z. B. Knie-Bänderriss, Gehirntumor und/oder Epilepsie). Die Pathologie wird an Bezugszeichen 324 in Metadaten umgesetzt.

Wie mittels (1) am Schritt 322 angedeutet, sind die eine oder mehreren Spulen und die Pathologie sind die Simulation relevant. Die Spule kann z. B. unterschiedlich viele Unterspulen (und/oder Kanäle) umfassen (z. B. haben Kopfspulen konventionellerweise zwischen 8 und 64 Kanäle und/oder Unterspulen). Die zu untersuchende Körperregion kann für die Auswahl des richtigen Ensembles an synthetischen Daten (und/oder die Auswahl der Gruppe von Atlanten im Schritt S104) nötig sein. Im Prinzip muss keine Einschränkung auf die Pathologie vorgenommen werden, wenn alle synthetischen Daten des Kopfes simuliert werden. Ist eine Pathologie vorher bekannt oder vermutet (z. B. "Tumor" und/oder "Epilepsie"), dann kann eine entsprechende Untermenge (insbesondere aus der Bibliothek) ausgesucht werden und nur diese für das Simulieren S106 verwendet werden. Die Einschränkung auf eine kleinere ausgewählte S104 Gruppe von Atlanten (und/oder ein kleineres Ensemble) spart direkt Rechenzeit, Prozessorressourcen und/oder Speicherressourcen.

An Bezugszeichen 326 passt der Nutzer (insbesondere physikalische und/oder technische) Parameter an (z. B. Auflösung, Bandweite und/oder TR). An Bezugszeichen 328 werden die angepassten (insbesondere physikalischen und/oder technischen) Parameter, die auch als Nutzereinstellungen bezeichnet werden können, als Metadaten umgesetzt.

Wie mittels (2) am Schritt 326 angedeutet, kann der Nutzer die (insbesondere physikalischen und/oder technischen) MRT-Parameter der Messung wählen. Die gewählten (insbesondere physikalischen und/oder technischen) MRT-Parameter können den Kontrast im Bild bestimmen (z. B., ob der Bildeindruck stärker durch die Relaxationszeit T1 oder T2* beeinflusst werden soll). Z. B. ist ein geeigneter Kontrast typischerweise durch radiologisches Wissen vorgegeben. Außerdem werden in einer MRT-Untersuchung typischerweise mehrere Bilder mit unterschiedlichen Kontrasten aufgenommen.

An Bezugszeichen 330 möchte der Nutzer das Messprotokoll beschleunigen. Hierzu kann der ein entsprechendes UI-Element aufgerufen.

Die Schritte 322, 326 und 330 können einem Ausführungsbeispiel des Schritts S102 des schematischen Verfahrens 100 der Fig. 1 entsprechen und/oder im Schritt S102 umfasst sein.

An Bezugszeichen 332 werden passende synthetische Atlanten mit Pathologien (z. B. von einem Rekonstruktionsrechner, kurz Recon-Rechner, als Ausführungsbeispiel der Rechenvorrichtung 200) ausgewählt (z. B. gemäß Verfahrensschritt S104) und zu einem Ensemble (und/oder eine ausgewählten S104 Gruppe von Atlanten) zusammengestellt. An Bezugszeichen 334 wird als Rechendaten ein Zähler-Array mit N Einträgen zusammengestellt. Der Zähler-Array wird initialisiert mit Güte[1..N]=0. Alternativ oder ergänzend werden Unterabtastungsfaktoren (und/oder Beschleunigungsfaktoren) R von 1 bis N an M Atlanten getestet. Das Array Güte[] kann für jeden Unterabtastungsfaktoren (und/oder Beschleunigungsfaktor) den prozentualen Anteil an Atlanten speichern, in welchen die Läsionen mit ausreichender Qualität (z. B. gemäß der Qualitätsmetrik) sichtbar sind.

Der Recon-Rechner kann dazu ausgebildet sein, (insbesondere Bild-) Rekonstruktionen auszuführen. Alternativ oder ergänzend kann der Recon-Rechner dazu ausgebildet sein, das erfindungsgemäße Verfahren auszuführen. Weiterhin alternativ oder ergänzend kann der Recon-Rechner ein Ausführungsbeispiel für die Rechenvorrichtung 200 sein.

An Bezugszeichen 346 wird als weitere Rechendaten das Ensemble mit M Atlanten zusammengestellt.

Wie an (3) am Schritt 332 angedeutet, kann angenommen werden, dass der Simulationsprozess (z. B. umfassend den Verfahrensschritt S106) und der Vergleich zur Ground Truth (z. B. im Verfahrensschritt S108 des Bestimmens der Qualitätsmetrik) auf dem Rekonstruktionsrechner (beispielsweise verkörpert durch das Simulationsmodul 208 und/oder die Rechenvorrichtung 200) durchgeführt wird, auf dem auch die KI-Rekonstruktion (insbesondere der geplanten medizinischen Aufnahme) später läuft. Dies ist jedoch nicht zwingend erforderlich. Alternativ oder ergänzend kann die Simulation der Daten auf einem anderen Rechner (beispielsweise der Rechenvorrichtung 200 mit einer Schnittstelle zu einem externen Simulationsmodul 208) erfolgen. Es kann nötig sein, die simulierten k-Raumdaten von den (z. B. tatsächlich) zum Einsatz kommenden Rekonstruktionsalgorithmen rekonstruieren zu lassen. Z. B. kann eine Interaktion mit dem Recon-Rechner (und/oder dem Simulationsmodul 206, und/oder der Rechenvorrichtung 200) spätestens zum Zeitpunkt der Bildrekonstruktion der simulierten k-Räume nötig sein.

An Bezugszeichen 336 wird ein Unterabtastungsfaktor (auch als Beschleunigungsfaktor bezeichnet) R=1...N gewählt. Der maximale Wert N des Unterabtastungsfaktors kann eine maximale Anzahl an Codierschritten umfassen.

An Bezugszeichen 338 in Fig. 3A fragt der Recon-Rechner (und/oder das Simulationsmodul 206, und/oder die Rechenvorrichtung 200) eine Unterabtastungsmaske für Nutzereinstellungen 328 mit Beschleunigung R beim Recon-Algorithmus an.

An Bezugszeichen 340 wird ein Zähler gestartet mit Treffer=0.

Die Unterabtastungsmaske kann, wie an Bezugszeichen 358 gezeigt, als Maske der zu behaltenden k-Raumzeilen in Rechendaten umgesetzt (und/oder gespeichert) werden.

An Bezugszeichen 342 werden die Atlanten in der ausgewählten Gruppe von Atlanten gezählt, z. B. als A=1..M (mit M der Anzahl der Atlanten im Ensemble 346 und/oder in der ausgewählten Gruppe 346 von Atlanten).

An Bezugszeichen 344 wird eine Auswahl des zu simulierenden Atlasses #A aus dem Ensemble 346 und/oder ausgewählten Gruppe 346 von Atlanten getroffen und, wie an Bezugszeichen 348 gezeigt, wird der ausgewählte Atlas als Rechendaten umgesetzt und/oder gespeichert.

Wie mit (4) am Schritt 344 angedeutet, können Atlanten (insbesondere aus dem Ensemble 346 und/oder ausgewählten Gruppe 346) der Reihe nach simuliert werden und/oder parallel auf mehreren (z. B. Recon-) Rechnern (und/oder Simulationsmodulen 206, und/oder Rechenvorrichtungen 200).

Wie in Fig. 3A angedeutet können die Schritte 332 bis 344 Teilschritte des Verfahrensschritts S104 des Auswählens der Gruppe von Atlanten umfassen, und/oder dem Verfahrensschritt S104 entsprechen.

An Bezugszeichen 350 wird eine Simulation eines unbeschleunigten MRT-Messprozesses gemäß den Nutzereinstellungen 328 für den Atlas 346 und den vollständigen k-Raum, wie durch die Rechendaten an Bezugszeichen 352 angedeutet, ausgeführt.

Wie mit (5) am Schritt 350 angedeutet, kann die Simulation über die im Atlas enthaltenen MRT-Parameter sowie die MRT-Einstellungen 328 des Nutzers erfolgen. Für die Simulation von MRT-Anwendungen werden konventionellerweise Bloch-Gleichungen oder Erweiterungen der Bloch-Gleichungen verwendet und/oder benötigt. Alternativ oder ergänzend kann (z. B. theoretisch) auch die komplette MRT-Aufnahme mitsimuliert werden (z. B. mit allen Verstärkerkennlinien und/oder Gradientenimperfektionen). Im Allgemeinen sollte die optionale komplett mitsimulierte MRT-Aufnahme aber nicht nötig sein.

An Bezugszeichen 354 wird eine konventionelle Rekonstruktion, insbesondere über FFT (und/oder ohne KI) durchgeführt, insbesondere anhand des vollständigen k-Raums 352. Wie an Bezugszeichen 356 angedeutet, wird dadurch eine Ground Truth (z. B. als erste Rekonstruktion) zum ausgewählten Atlas 346 als Rechendaten bereitgestellt.

Wie mit (6) am Schritt 354 angedeutet, wird ein unbeschleunigter und konventionell rekonstruierter Datensatz der Atlasdaten 346 in diesem Ausführungsbeispiel benötigt. Dies erleichtert den Vergleich mit dem beschleunigten Bild, da die Kontrastverhältnisse in beiden identisch sind.

An Bezugszeichen 360 wird der vollständiger k-Raum 352 unterabgetastet gemäß Maske 358. An Bezugszeichen 362 ist der unterabgetasteter k-Raum als Rechendaten in Fig. 3A umgesetzt und/oder gespeichert.

An Bezugszeichen 364 wird der unterabgetasteter k-Raum 362 in den KI-Recon-Algorithmus, z. B. auf dem Recon-Rechner (und/oder dem Simulationsmodul 206, und/oder der Rechenvorrichtung 200), geladen und rekonstruiert. An Bezugszeichen 366 wird das unterabgetastete Bild (z. B. als mindestens eine zweite Rekonstruktion) als Rechendaten bereitgestellt.

Die Schritte 350 bis 366 können Teilschritte des Verfahrensschritts S106 des Simulierens von mindestens zwei Rekonstruktionen für jeden Atlas aus der ausgewählten Gruppe von Atlanten umfassen, und/oder dem Verfahrensschritt S106 entsprechen.

An Bezugszeichen 368 in Fig. 3A wird ein Vergleich zwischen Ground Truth 356 zum Atlas und unterabgetastetem Bild 366 mit den Segmentierungsinformationen aus dem Atlas #A vorgenommen. Der Vergleich kann einen Signalunterschied, SSIM und/oder andere Bildmetriken umfassen.

Wie mit (7) am Schritt 368 angedeutet, können über die Segmentierungsangaben im Atlas Regionen von gesundem und krankhaftem Gewebe (z. B. als ROIs) bekannt sein. Die Ortsinformation (z. B. von gesundem und krankhaftem Gewebe) kann verwendet werden, um z. B. Signalunterschiede zwischen gesundem und krankem Gewebe zu berechnen, welche zwischen dem beschleunigtem 366 und unbeschleunigtem 358 Bild verglichen werden können. Alternativ oder ergänzend können direkt Signalunterschiede zwischen krankhaften Geweben der beschleunigten 366 und unbeschleunigten 358 Bilder vergleichen werden. Gemäß einem (mit jedem weiteren Ausführungsbeispiel kombinierbaren) Ausführungsbeispiel kann eine Normierung der Daten nötig sein, insbesondere falls innerhalb der Rekonstruktionsalgorithmen (kurz: Recon-Algorithmen), z. B. auf einem oder mehreren Recon-Rechnern (und/oder Simulationsmodulen 206, und/oder Rechenvorrichtungen 200) unterschiedlich skaliert wird. Beispielsweise kann auf das mittlere Signal des Bildes normiert werden.

An Bezugszeichen 370 werden in Fig. 3B überprüft, ob Läsionen mit ausreichend hohem Bildmetrikwert (z. B. Signalunterschied) im rekonstruiertem Bild 366 vorhanden sind. Falls ja, wird an Bezugszeichen 370-Y mit dem Schritt 372 des Zählerinkrementierens fortgefahren. Wie an Bezugszeichen 374 angedeutet, erhöht der Zähler bei einem Treffer den Zählerwert (und/oder Treffer) um eins (+1).

Wie mit (8) am Schritt 370 angedeutet, kann (und/oder muss) die Frage, wann ein Unterschied als "ausreichend" angesehen wird, (z. B. vom Hersteller des Simulations-Algorithmus und/oder des Tomographen) in einem passenden Prozess experimentell festgelegt werden. Alternativ oder ergänzend können harte (z. B. im Vorhinein festgelegte) Grenzwerte verwendet werden und/oder potentiell wiederum KI-Algorithmen oder Heuristiken verwendet werden, welche einen oder mehrere (z. B. harte) Grenzwerte abschätzen.

Falls die Läsionen nicht mit ausreichend hohem Bildmetrikwert (z. B. Signalunterschied) im rekonstruiertem Bild 366 vorhanden sind, wird an Bezugszeichen 370-N direkt (z. B. unter Auslassung des Schritts 372 des Inkrementieren des Zählers) mit dem Schritt 376 fortgefahren, in dem überprüft wird, ob alle Atlanten des Ensembles 346 (und/oder der ausgewählten Gruppe 346 von Atlanten) simuliert worden sind. Falls nicht, wird an Bezugszeichen 365-N eine Schleife zurück zum Schritt 342 der Zählung der Anzahl der Atlanten im Ensemble 346 (und/oder in der ausgewählten Gruppe 346) gebildet.

Falls alle Atlanten des Ensembles 346 (und/oder der ausgewählten Gruppe 346 von Atlanten) simuliert worden sind, wird an Bezugszeichen 376-Y fortgefahren mit dem Schritt 378 der Berechnung der prozentualen Anzahl (z. B. der Atlanten und/oder Rekonstruktionen), in der die Pathologien im mittels KI rekonstruierten Bild (und/oder in der mindestens eine zweite Rekonstruktion) im Vergleich zur Ground Truth 356 sichtbar waren.

An Bezugszeichen 380 können Rechendaten in der Form Güte[R]=Treffer/M bereitgestellt und/oder gespeichert werden.

Die Schritte 368 des Vergleichens zwischen unbeschleunigtem Bild 356 und beschleunigtem Bild 366 bis zum Schritt 380 der Bestimmung der Güte der (insbesondere simulierten) beschleunigten MRT-Messprozesse können Teilschritte des Verfahrensschritts S108 des Bestimmens der Qualitätsmetrik umfassen, und/oder dem Verfahrensschritt S108 entsprechen.

An Bezugszeichen 382 wird bestimmt, ob auf dem UI nur die Beschleunigungen angezeigt werden, für welche Güte[R]=1 und/oder ein anderer optimaler Wert der Qualitätsmetrik gilt. Güte[R]=1 kann für eine KI Rekonstruktion (und/oder die mindestens eine zweite Rekonstruktion) mit Unterabtastungsfaktor (und/oder Beschleunigungsfaktor) R einer korrekten Wiedergabe gemäß der Qualitätsmetrik von 100% aller Läsionen der simulierten Atlanten entsprechen.

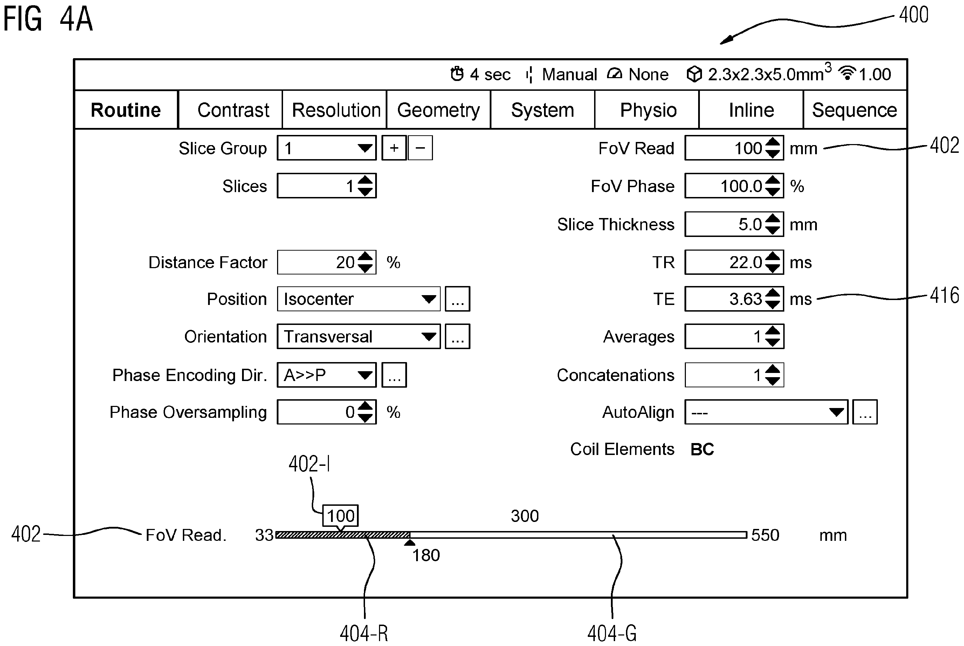

Wie mit (9) am Schritt 382 angedeutet, kann es eine Frage der Nutzerführung sein, ob nur Beschleunigungsfaktoren im UI erlaubt werden, welche alle Läsionen des Ensembles 346 (und/oder der ausgewählten Gruppe 346) korrekt wiedergegeben haben (z. B. gleichbedeutend mit Güte[R]=1), oder ob auch andere Beschleunigungsfaktoren erlaubt werden (z. B. gleichbedeutend mit Güte[R]<1; z. B. "roter Bereich" 404-R in einem Einstellungsbalken in Fig. 4A, und/oder Unterabtastungsfaktoren, für die nicht, und/oder zumindest nicht korrekt, alle Läsionen wiedergegeben werden im unterabgetasteten Bild 366). Beispielsweise kann es gemäß einer (z. B. Hersteller-) Philosophie nur Sinn machen, einen roten Bereich 404-R (z. B. in Fig. 4A) anzuzeigen, wenn dem Nutzer durch die Rechenvorrichtung 200 (z. B. verkörpert durch einen Computer) Vorschläge unterbreitet werden, wie ein aus dem roten Bereich gewählter Beschleunigungsfaktor so eingestellt werden kann, dass wiederum das komplette Ensemble 346 (und/oder die ausgewählte Gruppe 346 von Atlanten) korrekt wiedergegeben wird. Durch die hohe Abhängigkeit der einstallbaren Parameter der MRT-Messtechnik kann dies z.B. dadurch erzeugt werden (und/oder der Vorschlag umfassen), dass (z. B. andere) kontrastgebende Parameter variiert werden (z.B. Erhöhung eines Flipwinkels und/oder Änderung der Aufnahmebandweite).

Alternativ oder ergänzend kann die Rechenvorrichtung 200 (z. B. verkörpert durch den Computer) mit dem gewünschten Beschleunigungsfaktor andere (insbesondere physikalische und/oder technische) Parameter der Bildgebungssequenz variieren und/oder überprüfen, ob das Ensemble 346 (und/oder die ausgewählte Gruppe 346 von Atlanten) korrekt wiedergegeben wird. Durch die konventionellerweise hohe Anzahl an (insbesondere physikalischen und/oder technischen) Parametern (insbesondere für einen MRT-Scan) kann es (insbesondere ohne Vorauswahl einer, z. B. kleineren, Untergruppe von Parametern) unwahrscheinlich dass, dass das Variieren der anderen (insbesondere physikalischen und/oder technischen) Parameter effektiv und/oder effizient gelingt.

Falls nur bei Güte[R]=1 (und/oder einem optimaler Wert der Qualitätsmetrik) eine Anzeige des Unterabtastungsfaktors auf dem UI 382 erfolgen soll, wird an Bezugszeichen 382-Y mit dem Schritt 384 des Überprüfens, ob die Güte[R]<1 (und/oder der Wert der Qualitätsmetrik nicht optimal ist), fortgefahren. Falls keine Auswahl der Anzeige auf dem UI stattfinden soll, wird an Bezugszeichen 382-N mit der Überprüfung 386 fortgefahren, ob alle Unterabtastungen (und/oder Beschleunigungen) simuliert wurden. Die Überprüfung 386, ob alle Beschleunigungen simuliert wurden, wird ebenfalls vorgenommen, wenn das Überprüfen 384, ob Güte[R]<1 (und/oder der Wert der Qualitätsmetrik nicht optimal ist), an Bezugszeichen 384-N ein "nein" ergibt.

Wenn nicht alle Beschleunigungen an Bezugszeichen 386 simuliert worden sind, wird an Bezugszeichen 386-N eine Schleife zurück zum Schritt 336 der Bestimmung der Beschleunigungsfaktoren ausgeführt.

Wenn, wie an Bezugszeichen 386-Y gezeigt, alle Beschleunigungen simuliert wurden, und/oder, wie an Bezugszeichen 384-Y gezeigt, Güte[R]<1 zutrifft (und/oder der Wert der Qualitätsmetrik nicht optimal ist), wird im Ausführungsbeispiel des Verfahrens 100 der Figs. 3A und 3B an Bezugszeichen S112; S112' mit der Anzeige der nutzbaren Beschleunigungsfaktoren (und/oder Unterabtastungsfaktoren) auf dem UI gemäß einem Güte[]-Array fortgefahren.

Das Güte[]-Array in dem Ausführungsbeispiel der Figs. 3A und 3B trägt die Information, wie hoch der relative Anteil der gemäß der Qualitätsmetrik korrekt wiedergegebenen Läsionen in den mit einem Unterabtastungsfaktor (und/oder Beschleunigungsfaktor), z. B. dem Faktor R, unterabgetasteten Datensätzen (und/oder synthetischen Messdatensätzen) ist. Dies kann dem Nutzer im UI z. B. über einen farbcodierten Balken visualisiert werden, welcher den Bereich des Unterabtastungsfaktors (und/oder Beschleunigungsfaktors) z. B. linear aufsteigend von links nach rechts dargestellt abdeckt. Die Farbe grün kann beispielweise anzeigen, dass in diesem Bereich die Rekonstruktionen der Läsionen der Atlanten zu 100% (und/oder zu einem anderen, vom Nutzer definierten Anteil und/oder Wert) erfolgreich waren. Der sich anschließende beispielsweise rote Bereich kann anzeigen, dass die Rekonstruktionen in diesem Bereich unterhalb von 100% Erfolgsquote lagen (unter 100% und/oder unterhalb des durch den Nutzer definierten Anteils und/oder Werts), und/oder dass sich durch Änderung anderer Parameter eine Verbesserung erzielen lässt. Alternativ oder ergänzend kann das Güte[]-Array über einen Schieberegler, und/oder ein Nummerneingabefeld, mit Inkrement-und-Dekrement-Elementen visualisiert werden, in welchem der Beschleunigungsfaktor z. B. von links nach rechts linear ansteigend unterlegt ist. Die Bewegung des Schiebereglers kann den Beschleunigungsfaktor auswählen. Die obere Grenze des wählbaren Bereichs kann durch den Unterabtastungsfaktor (und/oder Beschleunigungsfaktor) gegeben sein, bis zu welchem die Rekonstruktionen der Läsionen der Atlanten zu 100% (und/oder einem anderen, vom Nutzer definierten Anteil und/oder Wert) erfolgreich waren.

An Bezugszeichen S114 in Fig. 3B kann der Nutzer aus den gebotenen Möglichkeiten auswählen.

Wie mit (10) am Schritt S114 angedeutet, kann der Nutzer aus den dargebotenen Beschleunigungsfaktoren einen gewünschten Unterabtastungsfaktor auswählen. Alternativ oder ergänzend kann die Rechenvorrichtung 200 (auch als Steuerungscomputer bezeichnet), z. B. selbständig, den höchsten oder höchstmöglichen (z. B. gemäß einem Qualitätskriterium und/oder Wert der Qualitätsmetrik) Beschleunigungsfaktor wählen.

Der Schritt S114 in Fig. 3B kann dem Schritt S114 des Empfangens eines Verifikationssignals entsprechen.

An Bezugszeichen 388 wird das Ausführungsbeispiel des Verfahrens 100 beendet, beispielsweise indem eine Verifikationsmessung durchgeführt S116 wird oder der MRT-Scanner zur Ausführung der medizinischen Aufnahme mit dem festgelegten Unterabtastungsfaktor gesteuert S118 wird.

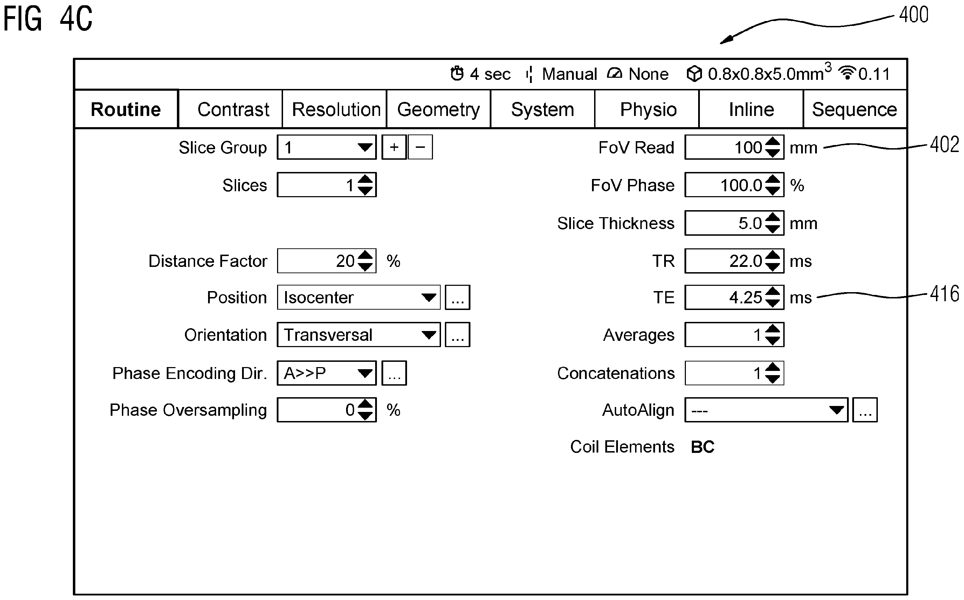

In Figs. 4A, 4B und 4C ist ein Beispiel einer Benutzeroberfläche (und/oder eines UIs) zur Benutzung im Rahmen der erfindungsgemäßen Technik am Beispiel einer MRT-Aufnahme (und/oder eines MRT-Scans) an einem MRT-Scanner (auch als MR-Tomograph, kurz MRT, bezeichnet) beschrieben.

Für MRT-Messungen können beispielsweise in der Größenordnung von 80 (insbesondere physikalischen und/oder technischen) Parametern eingestellt werden, welche die darzustellende Geometrie (und/oder anatomische Struktur), den Kontrast und die Messzeit beeinflussen. Viele der ca. 80 Parameter hängen voneinander ab (und/oder haben Interdependenzen), weshalb die Bedienung eines MRT üblicherweise viel Erfahrung voraussetzt. Es kann eine einfachere Steuerung mit Helferfunktionen implementiert werden, welche zulässige und/oder unzulässige (insbesondere physikalische und/oder technische) Parameter anzeigen und bei Änderungen der Parameter behilflich sein kann.

In Fig. 4A ist ein Balken 404-R; 404-G im unteren Bereich des Fensters gezeigt, der einen grünen Bereich 404-G die möglichen Einstellungen eines beispielhaften Parameters darstellt, welche ohne Änderung eines anderen Parameters realisierbar sind. In einem roten Bereich 404-R sind einstellbare Parameterwerte dargestellt, welche aber dazu führen, dass andere Parameter angepasst werden müssen. Im Beispiel der UI-Anzeige der Fig. 4A soll die Größe des darzustellenden Bereichs (und/oder Sichtfelds bzw. FoV Reads 402) in eine Raumrichtung (also ein Geometrieparameter) auf 100 mm gesetzt werden. Im Beispiel der Fig. 4A liegt der Wert 402-1 von 100 mm im roten Bereich 404-R des Einstellungsbalkens 404-R; 404-G.

Die Rechenvorrichtung 200 (und/oder der Computer) unterstützt im Ausführungsbeispiel der Figs. 4A, 4B und 4C den Nutzer mit einem Vorschlag, dass die Echozeit (als Beispiel eines Kontrastparameters) verlängert werden muss.

In Fig. 4B wird an Bezugszeichen 416 die vorgeschlagene Veränderung der Echozeit (TE) als beispielhafter anderer (insbesondere physikalischer und/oder technischer) Parameter angezeigt, um die Änderung des Sichtfelds (englisch: Field of View, FOV) entlang einer (z. B. logischen) MRT-spezifischen Raumrichtung (und/oder Auslese-Richtung; englisch: Read) zu erlauben. Der Vorschlag kann mittels der Bestätigungsschaltfläche 412 angenommen, oder mittels der Ablehnungsschaltfläche 414 verworfen werden.

In Fig. 4C wird eine beispielhafte Anzeige des UI gezeigt, nachdem der Vorschlag zur Änderung der Echozeit (TE) 416 bei Änderung des Sichtfelds (FoV Read) 402 durch den Nutzer akzeptiert wurde.

In Figs. 4A, 4B und 4C sind als weitere einstellbare (insbesondere physikalische und/oder technische) Parameter das Gesichtsfeld entlang einer weiteren (z.B. logischen) MRT-spezifischen Raumrichtung (und/oder Phasenkodierrichtung; englisch Phase), eine Schichtdicke (englisch: slice thickness) und eine Repetitionszeit (TR) angegeben.

Alternativ oder ergänzend kann es noch andere Möglichkeiten geben, die Änderung (z. B. des Sichtfelds) zu erreichen, z. B. eine Reduktion der Auflösung (als Beispiel eines Geometrieparameters) und/oder eine Anpassung der Bandweite (als Beispiel für einen Parameter, der das Signal-zu-Rauschen-Verhältnis, SNR, beeinflusst).

Die Änderungsvorschläge für einstellbare (insbesondere physikalische und/oder technische) Parameter können im Hintergrund über eine Suchfunktion gestartet werden. Z. B. kann die Rechenvorrichtung 200 (und/oder der Computer) im Hintergrund unterschiedliche Werte für die Echozeit (TE) einsetzen und eine Konsistenzcheck-Funktion der Sequenz aufrufen. Das Ergebnis der Konsistenzcheck-Funktion kann beobachtet werden, wenn weitere insbesondere physikalische und/oder technische) Parameter verändert werden.

Im Ausführungsbeispiel der Figs. 4A, 4B und 4C werden nur von einem Programmierer (insbesondere als einstallbar) hinterlegte (insbesondere physikalische und/oder technische) Parameter getestet. Eine Suche im kompletten Parameterraum ist in dem Ausführungsbeispiel prohibitiv.

Im Rahmen der erfinderischen Technik kann, wenn Läsionen eines Ensembles ab einem gewissen Beschleunigungsfaktor nicht mehr vollständig erhalten bleiben, der Bereich, wie in Fig. 4A an Bezugszeichen 404-R gezeigt, rot markiert werden. Wählt der Nutzer dann einen (z. B. Unterabtastungsfaktor-) Parameter aus dem (insbesondere roten) Bereich 404-R aus, kann ein Suchalgorithmus im Hintergrund prüfen, ob es (insbesondere physikalische und/oder technische) MRT-Parameter gibt, welche geändert werden können und zu einer Abbildung der Läsionen im Ensemble (und/oder in der ausgewählten Gruppe der Atlanten) führen. Nachteilig könnte je nach Ausführungsbeispiel sein, dass hierbei potentiell an vielen (insbesondere physikalischen und/oder technischen) Parametern Veränderungen vorgenommen werden müssen, wodurch sich der gewünschte Kontrast des Bildes ändern kann. Zusätzlich kann eine Rechenzeit durch viele unterschiedliche Parameterkombinationen in einen prohibitiven Bereich steigen (beispielsweise keine vorherige Einschränkung auf eine Untergruppe einstellbarer Parameter vorgenommen wird).

MRT-Protokolle sind aufgrund der (insbesondere physikalischen und/oder technischen) Parameter und deren Interdependenzen komplex zu optimieren (z. B. hinsichtlich Anzahl und Zusammenspiel von Relaxationszeiten, Repetitionszeiten, Bandweiten und/oder Spulenprofilen).

Gemäß einem Ausführungsbeispiel kann ein einfach verständliches Werkzeug zur Verfügung gestellt werden, um ungeübten Nutzern schnell die Auswirkungen einer Änderung der (insbesondere physikalischen und/oder technischen) Parameter und/oder des Unterabtastungsfaktors anzuzeigen. Somit kann die Dauer der Protokolloptimierung verkürzt und die Qualität der erzielten Bilder erhöht werden.

(Insbesondere physikalische und/oder technische) Parameter können aufgrund von Erfahrungswerten und/oder (konventionell seltener) aufgrund von theoretischen Abschätzungen einzelner Relaxationszeiten optimiert werden. Die Optimierung wird konventionell entweder an einem Phantom oder auch am Probanden durchgeführt bzw. verifiziert. Potentiell werden deshalb konventionellerweise mehrere Testmessungen benötigt, um herauszufinden, ob die ausgewählten und/oder getroffenen (insbesondere physikalischen und/oder technischen) Parameter zum gewünschten Kontrast und/oder SNR führen.

Die erfindungsgemäße Technik kann in einem Ausführungsbeispiel durch eine simulationsbasierte Protokolloptimierung (z. B. in der MRT) mittels eines UI-Elements mit zugehöriger Funktionalität unterstützt werden. Das Beispiel umfasst ein MRT, jedoch ist auch eine Anwendung auf andere Systeme denkbar (insbesondere umfassend Röhrenstrom, Rotationsgeschwindigkeit und/oder Rekonstruktionskernel bei einer CT).

Im MRT kann ein "Optimierungsmodus" für ein Protokoll aktiviert werden und der Nutzer kann eine ihn interessierende Anatomie (auch anatomische Struktur bezeichnet) aussuchen (z. B. Kopf und/ Knie). Es kann eine ganze Bibliothek von Anatomien (und/oder Atlanten) geben. Die Bibliothek kann (z. B. vom Hersteller) synthetisch erzeugte und/oder exemplarisch (insbesondere auch "vor Ort", z. B. am Einsatzort des MRT-Scanners) gemessene Parameterkarten der interessierenden Anatomien umfassen. Die gewählten Sequenzparameter können über eine physikalische Simulation (z. B. für MRT mittels Bloch-Gleichungen, gegebenenfalls mit Erweiterungen) auf die gewählte Anatomie angewendet werden. Dadurch kann ein synthetisches Bild entstehen, das den zu erwartenden Bildeindruck der Sequenzparameter darstellt. Der Simulationsprozess kann über zusätzliche Informationen (z. B. einen digitalen Zwilling, Digital Twin, des Scanners, welcher beispielsweise Prozessketten, Spulen und/oder Rauschquellen berücksichtigt) verbessert werden und ein noch realistischer Bildeindruck erzielt werden. Wird vom Nutzer ein Parameter in der Sequenz geändert, kann der Simulationsprozess erneut gestartet werden. Dadurch können die Änderungen direkt ersichtlich sein, und eine Sequenz kann z. B. hinsichtlich ihres Kontrasts bei Fettsättigung, SNR, T1w und/oder T2w schnell optimiert werden.

Die Anatomie (und/oder der Atlas) kann ebenfalls Informationen über Implantate enthalten. Die resultierten B0-Verzerrungen können zu Verzerrungen im MRT-Scan (und/oder bei CT zu Strahlaufhärtung) führen. Die Simulation der Bildgebungssequenz kann (z. B. sofort) die zu erwartende Bildqualität im Bereich des Implantats zeigen.

Alternativ oder ergänzend können Kontrastmittelanflutungen berücksichtigt werden. Weiterhin alternativ oder ergänzend können Bewegungen (z. B. des Patienten relativ zum Tomographen) während der Messung reproduzierbar simuliert werden.

Die Fälle (z. B. von Kontrastmittelanflutungen und/oder Bewegungen) bedeuten in der Realität komplexere Optimierungen bei konventionellen Verfahren nötig machen, da geeignete Probanden oder Patienten akquiriert werden müssen (insbesondere für reale Messungen). Im simulationsbasierten Ansatz ist kann die Optimierung auf eine Verifikationsmessung reduziert.

Des Weiteren ist es möglich, dass der Nutzer ROIs in die Anatomie (z. B. als Annotation in einem Atlas) einzeichnet und/oder Zielvorgaben macht (z. B. Optimierung auf hohes CNR zwischen zwei ROIs und, z. B. gleichzeitig, möglichst kurze Messzeit; SNR in einer Region und, z. B. gleichzeitig, möglichst geringe akustische Belastung). Ein Optimierungsprogramm kann geeignete Vorschläge (insbesondere zu den Zielvorgaben und/oder gehörig zu den ROIs) machen und die Vorschläge als synthetische Bilder präsentieren.

So kann ein schnellerer Eindruck der erzielten Bildqualität entstehen, der weniger bis keine Probanden und/oder Phantomuntersuchungen erfordert. Protokolle können auch über Remote Services (z. B. des Herstellers des Tomographen) optimiert und an die Gegebenheiten und/oder Rahmenbedingungen des Standorts des Tomographen angepasst werden. Alternativ oder ergänzend kann eine Protokolloptimierung von vergleichsweise unerfahrenen Mitarbeitern durchgeführt werden.

Die erfinderische Technik umfasst ein computer-implementiertes Verfahren zur Bestimmung eines Unterabtastungsfaktors für eine medizinische Aufnahme mittels eines Tomographen, welches ein Empfangen einer Anfrage hinsichtlich einer Planung der medizinischen Aufnahme mittels des Tomographen umfasst. Die Anfrage umfasst einen Hinweis auf einen Unterabtastungsfaktor. Eine Gruppe von Atlanten wird aus einer Bibliothek und basierend auf der empfangenen Anfrage ausgewählt. Jeder Atlas umfasst einen synthetischen Messdatensatz zu einer vorbestimmten Tomographen-Modalität. Für jeden Atlas aus der ausgewählten Gruppe von Atlanten werden zwei Rekonstruktionen simuliert zum Erstellen jeweils eines Bilddatensatzes. Eine erste Rekonstruktion basiert auf dem vollständigen synthetischen Messdatensatz. Eine zweite Rekonstruktion basiert auf einem Anteil des synthetischen Messdatensatzes entsprechend des empfangenen Hinweises auf den Unterabtastungsfaktor. Eine Qualitätsmetrik der zweiten Rekonstruktion wird bestimmt, wobei die simulierte zweite Rekonstruktion mit der simulierten ersten Rekonstruktion verglichen wird. Die bestimmte Qualitätsmetrik wird ausgegeben zum Festlegen des Unterabtastungsfaktors für die medizinische Aufnahme.

Soweit nicht bereits explizit beschrieben, können einzelne Ausführungsformen oder deren einzelne Aspekte und Merkmale, die in Bezug auf die Zeichnungen beschrieben sind, miteinander kombiniert oder ausgetauscht werden, ohne den Umfang der beschriebenen Erfindung einzuschränken oder zu erweitern, wenn eine solche Kombination oder ein solcher Austausch sinnvoll und im Sinne der vorliegenden Erfindung ist. Vorteile, die in Bezug auf eine bestimmte Ausführungsform der vorliegenden Erfindung oder in Bezug auf eine bestimmte Figur beschrieben sind, sind, wo immer dies zutrifft, auch Vorteile anderer Ausführungsformen der vorliegenden Erfindung.

Unabhängig vom grammatikalischen Geschlecht eines bestimmten Begriffes sind Personen mit männlicher, weiblicher oder anderer Geschlechteridentität mit umfasst.

## Patentansprüche

1. Computer-implementiertes Verfahren (100) zur Bestimmung eines Unterabtastungsfaktors für eine medizinische Aufnahme mittels eines Tomographen, umfassend die Schritte:
- Empfangen (S102) einer Anfrage hinsichtlich einer Planung einer medizinischen Aufnahme mittels eines Tomographen, wobei die Anfrage einen Hinweis auf einen Unterabtastungsfaktor umfasst;
- Auswählen (S104) einer Gruppe von Atlanten aus einer Bibliothek, wobei jeder Atlas in der Bibliothek einen synthetischen Messdatensatz zu einer vorbestimmten Tomographen-Modalität umfasst, und wobei das Auswählen (S104) der Gruppe von Atlanten auf der empfangenen (S102) Anfrage hinsichtlich der Planung der medizinischen Aufnahme mittels des Tomographen basiert;
- Simulieren (S106), für jeden Atlas aus der ausgewählten (S104) Gruppe von Atlanten, von mindestens zwei Rekonstruktionen zum Erstellen jeweils eines Bilddatensatzes, wobei eine erste Rekonstruktion auf dem vollständigen synthetischen Messdatensatz basiert, und wobei mindestens eine zweite Rekonstruktion auf einem Anteil des synthetischen Messdatensatzes entsprechend des mit der Anfrage empfangenen (S102) Hinweises auf den Unterabtastungsfaktor basiert;
- Bestimmen (S108) einer Qualitätsmetrik der mindestens einen zweiten Rekonstruktion, wobei das Bestimmen (S108) der Qualitätsmetrik ein Vergleichen der simulierten (S106) mindestens einen zweiten Rekonstruktion mit der simulierten (S106) ersten Rekonstruktion umfasst; und
- Ausgeben (S110) der bestimmten (S108) Qualitätsmetrik zum Festlegen des Unterabtastungsfaktors für die medizinische Aufnahme.

2. Verfahren (100) nach dem vorhergehenden Anspruch, wobei die Tomographen-Modalität ausgewählt ist aus der Gruppe:
- Magnetresonanztomographie, MRT;
- Computertomographie, CT; und

3. Verfahren (100) nach einem der vorhergehenden Ansprüche, wobei mindestens einer oder jeder Atlas aus der Gruppe der Atlanten eine dem synthetischen Messdatensatz zugeordnete Annotation, insbesondere eine Segmentierungsmaske, umfasst.

4. Verfahren (100) nach einem der vorhergehenden Ansprüche, ferner umfassend mindestens einen der Schritte:
- Ausgeben (S112) eines aufgrund der ausgegebenen (S110) Qualitätsmetrik festgelegten Unterabtastungsfaktors;
- Empfangen (S114) eines Verifikationssignal in Reaktion auf den, insbesondere ausgegebenen (S112), festgelegten Unterabtastungsfaktor; und
- Steuern (S118) des Tomographen zur Ausführung der medizinischen Aufnahme mit dem festgelegten Unterabtastungsfaktor.

5. Verfahren (100) nach einem der vorhergehenden Ansprüche, wobei die Qualitätsmetrik ausgewählt ist aus der Gruppe:
- Kontrast-Rausch-Verhältnis, CNR, insbesondere bei Fettsättigung;
- Signal-Rausch-Verhältnis, SNR;
- Transinformation;
- Differenzbild;
- Mittlere quadratische Abweichung, MSE; und
- Strukturelle Ähnlichkeit.

6. Verfahren (100) nach einem der vorhergehenden Ansprüche, wobei der synthetische Messdatensatz Messdaten in einem Frequenzraum umfasst, und wobei eine Unterabtastung gemäß dem Unterabtastungsfaktor ein Überspringen von Frequenzen umfasst.

7. Verfahren (100) nach einem der vorhergehenden Ansprüche, wobei der synthetische Messdatensatz Messdaten in einem Bildraum umfasst, und wobei eine Unterabtastung gemäß dem Unterabtastungsfaktor eine Wichtung und/oder ein Überspringen von Voxeln und/oder Pixeln und/oder Projektionen umfasst.

8. Verfahren (100) nach einem der vorhergehenden Ansprüche, wobei der Unterabtastungsfaktor größer als 1 ist, insbesondere wobei der Unterabtastungsfaktor im Wertebereich zwischen 2 und 100 liegt.

9. Verfahren (100) nach einem der vorhergehenden Ansprüche, wobei die Gruppe von Atlanten durch eine in jedem Atlas innerhalb der Gruppe erfasste anatomische Struktur und/oder Pathologie bestimmt ist.

10. Verfahren (100) nach einem der vorhergehenden Ansprüche, wobei die Anfrage hinsichtlich einer Planung einer medizinischen Aufnahme mittels eines Tomographen ferner einen Bereich von Interesse, einen Schwellenwert der Qualitätsmetrik, eine Vorgabe einer Auflösung und/oder eine Vorgabe hinsichtlich eines oder mehrerer Messparameter umfasst.

11. Verfahren (100) nach einem der vorhergehenden Ansprüche, wobei das Bestimmen (S108) der Qualitätsmetrik eine Abschätzung einer Größe einer anatomischen Struktur und/oder einer Anomalie in der mindestens einen zweiten Rekonstruktion und/oder der ersten Rekonstruktion umfasst.

12. Verfahren (100) nach einem der vorhergehenden Ansprüche, wobei beim Simulieren (S106) der mindestens zwei Rekonstruktionen ein Digitaler Zwilling des Tomographen verwendet wird.

13. Verfahren (100) nach einem der vorhergehenden Ansprüche, ferner umfassend den Schritt:
- Durchführen (S116) einer Verifikationsmessung mittels des Tomographen.

14. Verfahren (100) nach einem der vorhergehenden Ansprüche, ferner umfassend den Schritt:
- Ausgeben (S112'), aufgrund der ausgegebenen (S110) Qualitätsmetrik, einer Einstellung und/oder Änderung der Einstellung mindestens eines, insbesondere physikalischen und/oder technischen, Parameters für die medizinische Aufnahme mittels des Tomographen.

15. Rechenvorrichtung (200) zur Bestimmung eines Unterabtastungsfaktors für eine medizinische Aufnahme mittels eines Tomographen, umfassend:
- Eine Eingabeschnittstelle (202), die dazu ausgebildet ist, eine Anfrage hinsichtlich einer Planung einer medizinischen Aufnahme mittels eines Tomographen zu empfangen, wobei die Anfrage einen Hinweis auf einen Unterabtastungsfaktor umfasst;
- Ein Auswahlmodul (204), das dazu ausgebildet ist, eine Gruppe von Atlanten aus einer Bibliothek auszuwählen, wobei jeder Atlas in der Bibliothek einen synthetischen Messdatensatz zu einer vorbestimmten Tomographen-Modalität umfasst, und wobei das Auswählen der Gruppe von Atlanten auf der empfangenen Anfrage hinsichtlich der Planung der medizinischen Aufnahme mittels des Tomographen basiert;
- Ein Simulationsmodul (206) oder eine Schnittstelle zu einem Simulationsmodul (206), das dazu ausgebildet ist, für jeden Atlas aus der ausgewählten Gruppe von Atlanten mindestens zwei Rekonstruktionen zu simulieren zum Erstellen jeweils eines Bilddatensatzes, wobei eine erste Rekonstruktion auf dem vollständigen synthetischen Messdatensatz basiert, und wobei mindestens eine zweite Rekonstruktion auf einem Anteil des synthetischen Messdatensatzes entsprechend des mit der Anfrage empfangenen Hinweises auf den Unterabtastungsfaktor basiert;
- Ein Bestimmungsmodul (208), das dazu ausgebildet ist, eine Qualitätsmetrik der mindestens einen zweiten Rekonstruktion zu bestimmen, wobei das Bestimmen der Qualitätsmetrik ein Vergleichen der simulierten mindestens einen zweiten Rekonstruktion mit der simulierten ersten Rekonstruktion umfasst; und
- Eine Ausgabeschnittstelle (210), die dazu ausgebildet ist, die bestimmte Qualitätsmetrik zum Festlegen des Unterabtastungsfaktors für die medizinische Aufnahme auszugeben.

16. Rechenvorrichtung (200) nach dem unmittelbar vorhergehenden Anspruch, wobei die Rechenvorrichtung (200) ferner dazu ausgebildet ist, einen beliebigen oder jeden Schritt des Verfahrens (100) gemäß einem der Ansprüche 2 bis 14 auszuführen, oder im Zusammenhang mit dem Verfahren (100) gemäß einem der Ansprüche 2 bis 14 offenbarte Merkmale zu umfassen.

17. System zur Bestimmung eines Unterabtastungsfaktors für eine medizinische Aufnahme mittels eines Tomographen: umfassend:
- Eine Rechenvorrichtung (200) gemäß Anspruch 15 oder 16; und
- Einen Tomographen, mittels dem eine medizinische Aufnahme geplant wird.

18. Computerprogrammprodukt mit Programmelementen, die eine Rechenvorrichtung (200) veranlassen, die Schritte des Verfahrens zur Bestimmung eines Unterabtastungsfaktors für eine medizinische Aufnahme mittels eines Tomographen nach einem der vorhergehenden Verfahrensansprüche auszuführen, wenn die Programmelemente in einen Speicher der Rechenvorrichtung (200) geladen werden.
